(19) Europäisches Patentamt
European Patent Office
Office européen des brevets

(11) EP 2 180 418 B1

# (12) EUROPEAN PATENT SPECIFICATION

(45) Date of publication and mention of the grant of the patent:
**30.08.2023 Bulletin 2023/35**

(21) Application number: **09012959.4**

(22) Date of filing: **14.10.2009**

(51) International Patent Classification (IPC):
***G06F 17/18*** $^{(2006.01)}$ ***C12Q 1/6813*** $^{(2018.01)}$

(52) Cooperative Patent Classification (CPC):
(C-Sets available)
**C12Q 1/6813; G06F 17/18** (Cont.)

## (54) Determination of melting temperatures by equation-less methods

Bestimmung von Schmelztemperaturen mittels gleichungsfreien Verfahren

Détermination de températures de fusion par des procédés sans équation

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **17.10.2008 US 253676**

(43) Date of publication of application:
**28.04.2010 Bulletin 2010/17**

(73) Proprietors:
- **Roche Diagnostics GmbH**
  **68305 Mannheim (DE)**
  Designated Contracting States:
  **DE**
- **F. Hoffmann-La Roche AG**
  **4070 Basel (CH)**
  Designated Contracting States:
  **AT BE BG CH CY CZ DK EE ES FI FR GB GR HR HU IE IS IT LI LT LU LV MC MK MT NL NO PL PT RO SE SI SK SM TR**

(72) Inventor: **Kurnik, Ronald T.**
**Foster City, CA 94404 (US)**

(56) References cited:
**WO-A1-97/46714**
**WO-A1-2010/099461**
**US-A1- 2005 233 335**
**US-A1- 2009 037 117**

- **Idaho Technology Inc.: "LightCycler Software version 3.5 Users Manual", User manual, March 2001 (2001-03), XP055408587, Retrieved from the Internet: URL:http://www.biofiredefense.com/pdfs/LightCycler/LCDA_v3.5_Manual.pdf [retrieved on 2017-09-21]**
- **CHEN Y J ET AL: "DNA sequencing by denaturation: Principle and thermodynamic simulations", ANALYTICAL BIOCHEMISTRY, vol. 384, no. 1, 7 October 2008 (2008-10-07), pages 170-179, XP025679505, ISSN: 0003-2697, DOI: 10.1016/J.AB.2008.09.048 [retrieved on 2008-11-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6813, C12Q 2527/107, C12Q 2537/165**

**Description**

BACKGROUND OF THE INVENTION

The present invention relates generally to processing data representing melt characteristics of oligonucleotides, and more particularly to systems and methods for determining one or more melting temperatures for a DNA sample based on melt curve data.

Determination of DNA melting temperatures, usually performed directly after a PCR experiment, is an important method to distinguish genotypes. For example, recently discussed in the literature is the use of examining the KRAS gene to determine which patients might be candidates for a treatment for non-small cell lung cancer. Patients whose KRAS gene is of a wild-type would benefit from the treatment, whereas if the patient had a mutant variation of this gene, the treatment would be of no benefit. As these treatments often have major side effects, it is very important to determine the patient's correct genotype. The use of a melting KRAS assay can help distinguish the genotype of the patient. Therefore, it is desirable to provide systems and methods that accurately and efficiently determine the melting temperature of a DNA sample.

Herein, WO 97/46714 discloses methods for analyzing melting curves and quantifying the amount of DNA present. For quantification, in a first step a first derivative is calculated and plotted in a respective dF/dT v. T depiction and a baseline is subtracted from the plot. Finally, nonlinear regressions are applied to Gaussians using mean, standard deviation and height of peak as the fit parameters.

BRIEF SUMMARY OF THE INVENTION

Disclosed herein are systems and methods for determining melting temperatures, Tm, for DNA based on melt curve data. Herein, numerical determinations of the first derivatives of a melt curve data set are made. A model function, such as a Gaussian Mixture Model (GMM) function, with parameters determined using a Levenberg-Marquardt (LM) regression process is used to find an approximation to the first derivative curve. The maximum values of the numerically determined first derivative values are used as initial conditions for parameters of the model function. The determined parameters provide fractional melting temperature values, which can be returned, for example, displayed or otherwise used for further processing.

According to one aspect of the present invention, a computer-implemented method of determining a melting temperature, Tm, of DNA is provided. The method includes receiving a dataset representing a melt curve for a DNA sample, the dataset including a plurality of data points each having a pair of coordinate values. The method also includes numerically determining first derivative values for data points of the melt curve, determining a baseline for the first derivative values, subtracting out the baseline from the first derivative values to produce modified first derivative values, and determining whether the modified first derivative values include one or two or, optionally, three or four maximum values. The method also includes if it is determined that the modified first derivative values include a single maximum value, calculating an approximation of a curve that fits the modified first derivative values by applying a Levenberg-Marquardt (LM) regression process to a first Gaussian Mixture Model function, wherein said parameters include initial conditions, wherein the single maximum value is used as an initial condition for a first parameter, and wherein the first Gaussian Mixture Model function is:

$$GMM_1 = Exp(-a_1) \cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value. Further, the method comprises determining whether the first derivative values include a shoulder value proximal to the first maximum value by calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks; determining whether a shoulder value is present based on whether the height of the lower of said two peaks is at least a higher than a given percentage of the higher of said two peaks. The method further comprises outputting said maximum value, wherein said first maximum value represents a first melting temperature Tm1 of the DNA sample; and if it has been detected that a shoulder value is present, outputting said shoulder value, the shoulder value representing a second melting temperature Tm2 of the DNA sample. In yet another aspect of the method if it is determined that the modified first derivative values include three maximum values an approximation of a curve that fits the modified first derivative values is calculated by applying a Levenberg-Marquardt (LM) regression process to a third Gaussian Mixture Model function, wherein said parameters include initial condition, wherein the three maximum values are used as an initial conditions for the parameters, and wherein the Gaussian Mixture Model function is:

$$GMM_3 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right)$$

wherein $\mu_1$ is the first parameter, $\mu_2$ is the second parameter, $\mu_3$ is the third parameter and wherein $a_1$, $\sigma_1$, $a_2$, $\sigma_2$, $a_3$ and $\sigma_3$ are additional parameters; wherein the value of the first parameter $\mu_1$, the second parameter $\mu_2$, and the third parameter $\mu_3$ determined by the Levenberg-Marquardt regression process represents a first, second and third maximum value; and wherein the method further comprises outputting said first, second and third maximum values, wherein said first, second and third maximum values represent a first, second and third melting temperature Tm1, Tm2 and Tm3 of the DNA sample. In yet another aspect of the method if it is determined that the modified first derivative values include four maximum values an approximation of a curve that fits the modified first derivative values is calculated by applying a Levenberg-Marquardt (LM) regression process to a fourth Gaussian Mixture Model function, wherein said parameters include initial conditions, wherein the four maximum values are used as an initial condition for the parameters, and wherein the fourth Gaussian Mixture Model function is:

$$GMM_4 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right) + Exp(-a_4)\cdot Exp\left(-\frac{1}{2}\left(\frac{x-\mu_4}{\sigma_4}\right)^2\right)$$

wherein $\mu_1$ is the first parameter, $\mu_2$ is the second parameter, $\mu_3$ is the third parameter $\mu_4$ is the fourth parameter and wherein $a_1$, $\sigma_1$, $a_2$, $\sigma_2$, $a_3$, $\sigma_3$, $a_4$ and $\sigma_4$ are additional parameters; wherein the value of the first parameter $\mu_1$, the second parameter $\mu_2$, the third parameter $\mu_3$ and the fourth parameter $\mu_4$ determined by the Levenberg-Marquardt regression process represents a first, second, third and fourth maximum value; and wherein the method further comprises outputting said first, second, third and fourth maximum values, wherein said first, second, third and fourth maximum values represent a first, second, third and fourth melting temperature Tm1, Tm2 Tm3 and Tm4 of the DNA sample.

According to another aspect of the present invention, a computer readable medium is provided that stores code for controlling a processor to determine a melting temperature, Tm, of DNA. The stored code includes instructions to receive a dataset representing a melt curve for a DNA sample, the dataset including a plurality of data points each having a pair of coordinate values, to numerically determine first derivative values for data points of the melt curve, to determine a baseline for the first derivative values, to subtract out the baseline from the first derivative values to produce modified first derivative values, and to determine whether the modified first derivative values include one or two or, optionally, three or four maximum values. The code also includes instructions if it is determined that the modified first derivative values include a single maximum value, to calculate an approximation of a curve that fits the modified first derivative values by applying a Levenberg-Marquardt (LM) regression process to a first Gaussian Mixture Model function, wherein said parameters include initial conditions, wherein the single maximum value is used as an initial condition for a first parameter, and wherein the first Gaussian Mixture Model function is:

$$GMM_1 = Exp(-a_1) \cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value. Further, the code provides instructions to determine whether the first derivative values include a shoulder value proximal to the first maximum value by calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks; and determining whether a shoulder value is present based on whether the height of the lower of said two peaks is at least a higher than a given percentage of the higher of said two peaks. The code further comprises instructions to output the first maximum value, wherein said first maximum value represents a first melting temperature Tm1 of the DNA sample; and if it has been detected that a shoulder value is present, outputting said shoulder value, the shoulder value representing a second melting temperature Tm2 of the DNA sample. In yet another aspect the code comprises instructions that if it is determined that the modified first derivative values include three maximum values an approximation of a curve that fits the modified first derivative values is calculated by applying a Levenberg-Marquardt (LM) regression process to a third Gaussian Mixture Model function, wherein said parameters include initial condition, wherein the three maximum values are used as an initial conditions for the parameters, and wherein the third Gaussian Mixture Model function is:

$$GMM_3 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right)$$

wherein $\mu_1$ is the first parameter, $\mu_2$ is the second parameter, $\mu_3$ is the third parameter and wherein $a_1$, $\sigma_1$, $a_2$, $\sigma_2$, $a_3$ and $\sigma_3$ are additional parameters; wherein the value of the first parameter $\mu_1$, the second parameter $\mu_2$, and the third parameter $\mu_3$ determined by the Levenberg-Marquardt regression process represents a first, second and third maximum value; and wherein the method further comprises outputting said first, second and third maximum values, wherein said first, second and third maximum values represent a first, second and third melting temperature Tm1, Tm2 and Tm3 of the DNA sample. In yet another aspect the code comprises instructions that if it is determined that the modified first derivative values include four maximum values an approximation of a curve that fits the modified first derivative values is calculated by applying a Levenberg-Marquardt (LM) regression process to a fourth Gaussian Mixture Model function, wherein said parameters include initial condition, wherein the four maximum values are used as an initial conditions for the parameters, and wherein the fourth Gaussian Mixture Model function is:

$$GMM_4 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right) + Exp(-a_4)\cdot Exp\left(-\frac{1}{2}\left(\frac{x-\mu_4}{\sigma_4}\right)^2\right)$$

wherein $\mu_1$ is the first parameter, $\mu_2$ is the second parameter, $\mu_3$ is the third parameter $\mu_4$ is the fourth parameter and wherein $a_1$, $\sigma_1$, $a_2$, $\sigma_2$, $a_3$, $\sigma_3$, $a_4$ and $\sigma_4$ are additional parameters; wherein the value of the first parameter $\mu_1$, the second parameter $\mu_2$, the third parameter $\mu_3$ and the fourth parameter $\mu_4$ determined by the Levenberg-Marquardt regression process represents a first, second, third and fourth maximum value; and wherein the method further comprises outputting said first, second, third and fourth maximum values, wherein said first, second, third and fourth maximum values represent a first, second, third and fourth melting temperature Tm1, Tm2 Tm3 and Tm4 of the DNA sample. In another aspect of the computer readable medium the instructions to determine a first maximum value include instructions to apply a windowing process to the modified first derivative values, wherein the windowing process also determines one or more additional initial conditions for the Gaussian Mixture Model function parameters.

According to yet another aspect of the present invention, a kinetic Polymerase Chain Reaction (PCR) system is provided that includes a kinetic PCR analysis module adapted to generate a melt curve dataset representing a DNA melt curve, the dataset including a plurality of data points, each having a pair of coordinate values, and an intelligence module adapted to process the melt curve dataset to determine a Tm value. The intelligence module determines a Tm value by numerically determining first derivative values for data points of the melt curve, by determining a baseline for the first derivative values, by subtracting out the baseline from the first derivative values to produce modified first derivative values, and by determining whether the modified first derivative values include one or two or, optionally, three or four maximum values. The intelligence module if it is determined that the modified first derivative values include a single maximum value, calculates an approximation of a curve that fits the modified first derivative values by applying a Levenberg-Marquardt (LM) regression process to a first Gaussian Mixture Model function, wherein said parameters include initial conditions, and wherein the single maximum value is used as an initial condition for a first parameter, and wherein the first Gaussian Mixture Model function is:

$$GMM_1 = Exp(-a_1)\cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value. The intelligence module is further adapted to determine whether the first derivative values include a shoulder value proximal to the first maximum value by calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks and determining whether a shoulder value is present based on whether the height of the lower of said two peaks is at least a higher than a given percentage of the higher of said two peaks. The intelligence module is further adapted to outputting said first maximum value, wherein said first maximum value represents a first melting temperature Tm1 of the DNA sample; and if it has been detected that a shoulder value is present, outputting said shoulder value, the shoulder value representing a second melting temperature Tm2 of the DNA sample.

In certain aspects, if it is determined that the modified first derivative values include three maximum values an approximation of a curve that fits the modified first derivative values is calculated by applying a Levenberg-Marquardt (LM) regression process to a third Gaussian Mixture Model function, wherein said parameters include initial condition, wherein the three maximum values are used as an initial conditions for the parameters, the third Gaussian Mixture Model function is:

$$GMM_3 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right)$$

wherein $\mu_1$ is the first parameter, $\mu_2$ is the second parameter, $\mu_3$ is the third parameter and wherein $a_1$, $\sigma_1$, $a_2$, $\sigma_2$, $a_3$ and $\sigma_3$ are additional parameters; wherein the value of the first parameter $\mu_1$, the second parameter $\mu_2$, and the third parameter $\mu_3$ determined by the Levenberg-Marquardt regression process represents a first, second and third maximum value; and wherein the method further comprises outputting said first, second and third maximum values, wherein said first, second and third maximum values represent a first, second and third melting temperature Tm1, Tm2 and Tm3 of the DNA sample. In certain aspects, if it is determined that the modified first derivative values include four maximum values an approximation of a curve that fits the modified first derivative values is calculated by applying a Levenberg-Marquardt (LM) regression process to a fourth Gaussian Mixture Model function, wherein said parameters include initial conditions, wherein the four maximum values are used as an initial condition for the parameters, and wherein the fourth Gaussian Mixture Model function is:

$$GMM_4 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right) + Exp(-a_4)\cdot Exp\left(-\frac{1}{2}\left(\frac{x-\mu_4}{\sigma_4}\right)^2\right)$$

wherein $\mu_1$ is the first parameter, $\mu_2$ is the second parameter, $\mu_3$ is the third parameter, $\mu_4$ is the fourth parameter and wherein $a_1$, $\sigma_1$, $a_2$, $\sigma_2$, $a_3$, $\sigma_3$, $a_4$ and $\sigma_4$ are additional parameters; wherein the value of the first parameter $\mu_1$, the second parameter $\mu_2$, the third parameter $\mu_3$ and the fourth parameter $\mu_4$ determined by the Levenberg-Marquardt regression process represents a first, second, third and fourth maximum value; and wherein the method further comprises outputting said first, second, third and fourth maximum values, wherein said first, second, third and fourth maximum values represent a first, second, third and fourth melting temperature Tm1, Tm2 Tm3 and Tm4 of the DNA sample. In another aspect of the system the intelligence module determines a first maximum value by applying a windowing process to the modified first derivative values, wherein the windowing process also determines one or more additional initial conditions for the Gaussian Mixture Model function parameters.

Reference to the remaining portions of the specification, including the drawings and claims, will realize other features and advantages of the present invention. Further features and advantages of the present invention, as well as the structure and operation of various embodiments of the present invention, are described in detail below with respect to the accompanying drawings. In the drawings, like reference numbers indicate identical or functionally similar elements.

BRIEF DESCRIPTION OF THE DRAWINGS

FIG. 1 illustrates an example of a melt curve.
FIG. 2 illustrates a first derivative curve for the melt curve of FIG. 1.
FIG. 3 shows the derivative curve of FIG. 2 after baseline subtraction.
FIG. 4 illustrates a process for determining melting temperature(s) according to one implementation.
FIG. 5 illustrates a Melting Temperature computation process according to one implementation.
FIG. 6 illustrates a shoulder detection process according to one implementation.
FIGS. 7a and 7b show an example of a raw data curve and a melting curve, respectively, in the case of a single melting peak.

FIGS. 8a and 8b show an example of a raw data curve and a melting curve, respectively, in the case of a two melting peaks.

FIGS. 9a and 9b show an example of a raw data curve and a melting curve, respectively, in the case of a two melting peaks.

FIGS. 10a and 10b show an example of a raw data curve and a melting curve, respectively, in the case of a single melting peak plus a shoulder.

FIGS. 11a and 11b show an example of a raw data curve and a melting curve, respectively, in the case of a single melting peak plus a shoulder.

FIG. 12 shows a general block diagram explaining the relation between software and hardware resources that can be used to implement the processes and system of the invention.

FIG. 13 illustrates the interaction between a thermocycler device and a computer system.

DETAILED DESCRIPTION OF THE INVENTION

Disclosed herein are systems and methods for determining melting temperatures, Tm, for DNA by analyzing data representing a melt curve. Herein, numerical determinations of the first derivatives of a melt curve data set are made. A Gaussian Mixture Model (GMM) function with parameters determined using a Levenberg-Marquardt (LM) regression process is used to find an approximation to the first derivative curve. The maximum values of the numerically determined first derivative values are used as initial conditions for parameters of the GMM function. The determined parameters provide fractional melting temperature, Tm, values. The Tm value(s) are then returned and may be displayed or otherwise used for further processing.

One example of a melt curve in the context of a PCR process is shown in FIG. 1. As shown in FIG. 1, data for a typical melt curve can be represented in a two-dimensional coordinate system, for example, with temperature defining the x-axis and an indicator of accumulated polynucleotide defining the y-axis. Typically, the indicator of accumulated polynucleotide is a fluorescence intensity value as the use of fluorescent markers is perhaps the most widely used labeling scheme. However, it should be understood that other indicators may be used depending on the particular labeling and/or detection scheme used. Examples of other useful indicators of accumulated signal include luminescence intensity, chemiluminescence intensity, bioluminescence intensity, phosphorescence intensity, charge transfer, voltage, current, power, energy, temperature, viscosity, light scatter, radioactive intensity, reflectivity, transmittance and absorbance.

**General Process Overview**

Consider a typical melt curve as shown in FIG. 1. It is desired to obtain one or more melting temperatures from the data represented in FIG 1. Herein, a process 100 for determining a melting temperature can be described briefly with reference to FIG. 4. In step 110, an experimental data set representing the melt curve is received or otherwise acquired. An example of a plotted melt curve data set is shown in FIG. 1, where the y-axis and x-axis represent fluorescence intensity and temperature, respectively, for a melt curve. In certain aspects, the data set should include data that is continuous and equally spaced along an axis.

In the case where process 100 is implemented in an intelligence module (e.g., processor executing instructions) resident in a PCR device such as a thermocycler, the data set may be provided to the intelligence module in real time as the data is being collected, or it may be stored in a memory unit or buffer and provided to the intelligence module after the experiment has been completed. Similarly, the data set may be provided to a separate system such as a desktop computer system or other computer system, via a network connection (e.g., LAN, VPN, intranet, Internet, etc.) or direct connection (e.g., USB or other direct wired or wireless connection) to the acquiring device, or provided on a portable medium such as a CD, DVD, floppy disk or the like. In certain aspects, the data set includes data points having a pair of coordinate values (or a 2-dimensional vector). For melt data, the pair of coordinate values typically represents the temperature and the fluorescence intensity value. After the data set has been received or acquired in step 110, the data set may be analyzed to determine the melting temperature(s).

In step 120, the data is numerically processed to determine derivative values. The melting temperatures for these curves is obtained by finding the (fractional) temperature value (x-axis) corresponding to the maximum of the first derivative (y-axis) of the fluorescence intensity with respect to temperature. Using the data shown in FIG. 1, corresponding graphs for the first derivative is shown in FIG. 2. A baseline subtraction is performed on the derivative curve data to produce modified derivative data. Herein, baseline subtraction may be performed by first defining "MedianLeft" as the median of the fluorescent values of the first five points in FIG 2, then defining "MedianRight" as the median of the fluorescent values of the last five points in FIG. 2. A straight line is then defined connecting the "MedianLeft" point (x, y) in FIG. 2 with the "MedianRight" point (x, y). The slope and intercept of this straight line is then subtracted from all coordinate pairs. FIG. 3 shows the derivative curve after baseline subtraction.

Derivatives may be obtained by use of the Savitzky-Golay (SG) method. [See A. Savitzky and Marcel J.E.

Golay (1964). Smoothing and Differentiation of Data by Simplified Least Squares Procedures. Analytical Chemistry, 36: 1627-1639 and Press, W.H., et al. "Numerical Recipes in C, 2nd Ed.," Savitzky-Golay Smoothing Filters, Section 14.8, 650-655.]. A SG-2-2-2 configuration (meaning 2 points to the left, 2 points to the right, and 2nd degree polynomial) may be used to calculate the first derivative of the raw melting data curve. In general, other configurations of the SG method may be used, for example, SG-1-1-2 to SG-50-50-2. More generally, SG-x-y-z may be used where x and y are numbers from 1 to 50 and z is a number from 1 to 5.

**Scale Invariant Forms of derivatives**

Alternative methods may be used to calculate derivatives in order to allow the melting temperature to be scale-invariant. Scale invariant means that if fluorescence values are multiplied by a constant, the resulting Tm value(s) are unchanged.

According to one method, the fluorescence value is divided by the mean fluorescence value before calculation of the derivative melting curve, e.g., y is replaced by:

$$y / y_{mean}, \text{ where } y_{mean} = \tfrac{1}{n}\sum_{i=1}^{n} y_i . \qquad (1)$$

According to another method, the fluorescence value is divided by the (maximum fluorescence - minimum fluorescence) before calculation of the derivative melting temperatures.

According to another method, the derivative of the fluorescence value is divided by the fluorescence value before calculation of the melting temperatures.

According to yet another method, the derivative of the fluorescence value is divided by the mean derivative of the fluorescence value before calculation of the melting temperatures.

According to still another method, the derivative of the fluorescence value is divided by the (maximum-minimum) derivative of the fluorescence value before calculation of the melting temperatures.

Returning to FIG. 4, in step 130, a number of peaks in the first derivatives data is determined. A local maximization process is used to determine zero, one, two three or four peaks in the first derivatives data as will be discussed in more detail below. The raw derivatives data may be used, or the baseline subtracted derivatives data may be used.

Fractional Tm values are determined in step 140. To find the maximum values of the curves, for example as shown in FIGS. 2 or 3, a Gaussian Mixture Model is fit to the data. The mean of the Gaussian Mixture Model corresponds to the maximum, and hence the Tm value. A curve fit is done by calculating an approximation of a curve that fits the determined first derivative values, or modified (baseline subtracted) derivative values, by applying a regression process to a Gaussian Mixture Model function to determine one or more parameters of the function. Herein, a Levenberg-Marquardt regression process is used. For the case of a single peak, a Gaussian Mixture Model for one peak is used as shown in equation (2). If two peaks are present, a Gaussian Mixture Model for two peaks is used as shown in equation (3). If three peaks are present, a Gaussian Mixture Model for three peaks can be used as shown in equation (4). If four peaks are present, a Gaussian Mixture Model for four peaks can be used as shown in equation (5). The regressed values for the coefficients $\mu_1$ or ($\mu_1$, $\mu_2$) correspond to the Tm values for one and two peaks respectively. Gaussian Mixture Models are used herein, rather than taking additional derivatives to find the maximum, as higher order derivatives (3rd, and 4th) can become unstable.

$$GMM_1 = Exp(-a_1)\cdot Exp\left(-\frac{1}{2}\left(\frac{x-\mu_1}{\sigma_1}\right)^2\right) \qquad (2)$$

$$GMM_2 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) \qquad (3)$$

$$GMM_3 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right) \quad (4)$$

$$GMM_4 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right) + Exp(-a_4) \cdot Exp\left(-\frac{1}{2}\left(\frac{x-\mu_4}{\sigma_4}\right)^2\right) \quad (5)$$

It should be appreciated that other models/functions, which are not part of the invention, could be used instead of a Gaussian Mixture Model as would be apparent to one skilled in the art. Examples of other models include Beta, Binomial, Cauchy, Chi, ChiSquare, Exponential, Extreme Value, FRatio, Gamma, Gumbel, Laplace, Logistic, Maxwell, Pareto, Rayleigh, StudentT, and Weibull models.

It is to be understood that the above embodiment can be applied with first derivative data comprising more than two peaks. In this case, the initial estimates (regressed values) for the coefficients $\mu_1, \mu_2, \mu_3, \mu_4$ by the local maximum corresponds to the final melting temperature.

The Levenberg-Marquardt (LM) method is used to curve fit equation (2) (or equation (3), equation (4), equation (5)). Details of this method can be found in the reference [Moré, J.J., "The Levenberg-Marquardt Algorithm, Implementation and Theory," Numerical Analysis, ed. Watson, G.A. Lecture Notes in Mathematics 630, Springer-Verlag, 1977]. It should be appreciated that other regression methodsas are well known, which are not part of the invention, may be used. In general, the LM regression method includes an algorithm that requires various inputs and provides output. In one aspect, the inputs include a data set to be processed, a function (*e.g.*, Gaussian Mixture Model) that is used to fit the data, and an initial guess for the parameters or variables of the function. The output includes a set of one or more parameters for the function that minimizes the sum of the squares of the distance between the function and the data set. It should be appreciated that other regression processes, which are not part of the invention, as would be apparent to one skilled in the art may be used.

One feature of the Levenberg-Marquardt method is that it requires good estimates of the parameter values prior to performing the regression. For the parameters $a_1$ (or $a_1$, $a_2$, $a_3$, $a_4$ ) and $\sigma_1$ (or $\sigma_1$, $\sigma_2$, $\sigma_3$, $\sigma_4$) the initial conditions can be set equal to a constant (e.g., 1 or 2) in all cases. These parameters are generally not sensitive and will generally converge regardless of the initial conditions used. The parameters $\mu_1$ (or $\mu_1$, $\mu_2$, $\mu_3$, $\mu_4$) may require more accurate initial conditions that should be determined for each curve. Further, a windowing method may be used to calculate initial conditions for the parameters $\mu_1$ (or $\mu_1$, $\mu_2$, $\mu_3$, $\mu_4$) as described in more detail below.

In optional step 150, one or more expert system checks are performed to assess whether the results are valid as will be discussed in more detail below. For example, if implemented, an expert system check may determine that the determined result is invalid.

In step 160, the Tm value(s) are returned, e.g., for display or further processing. Graphical displays may be rendered with a display device, such as a monitor screen or printer, coupled with the system that performed the analysis of FIG. 4, or data may be provided to a separate system for rendering on a display device.

The $R^2$ statistic and/or confidence (e.g., 95% confidence) intervals may be calculated for the $GMM_1$, $GMM_2$, $GMM_3$ $GMM_4$ parameters. These values assess the quality of the curve fit and may be used in an Expert System (described below) to help determine whether the calculated Tm values are valid, invalid, or zero (no sample present). These values may also be displayed in step 160.

**Determination of Maximum in Curves**

A windowing process may be used on the data set to determine initial conditions for the parameters $\mu_1$, $\mu_2$, $\mu_3$, $\mu_4$. The following windowing process is described for a first derivative comprising up to two peaks. Nevertheless, as mentioned above, it is to be understood that this windowing process can be applied to first derivative data presenting up to four peaks. The windowing process may search for potential local maxima by using the following procedure:

1. Starting at the first point, examine the first several (e.g., five) points (points 1-5) of the data set.

2. If the middle y-Point is not the maximum in these five points, then there is no potential maximum in these five points. If the middle y-point is the maximum of these five points and it has a value greater than 0 (to avoid adding the middle points of a longer sequence of points with an exact value of 0 into the set of potential maxima), then there is a potential maximum. Add this point to the set of potential maxima **S.**

3. Advance the sliding window by one point (e.g., now points 2-6), and repeat the process described in item 2, again accepting only maxima at index 3 of these five points. Continue this process throughout the entire data set.

4. Examine the result set **S** of possible maxima, representing the set of possible maxima at index 3, and find the maximum data point in this set **S** of possible maxima (**Smax**).

5. If **Smax** is equal or less than a MaxNoise Input Parameter ( a noise parameter that may be input by a user, or automatically determined), there is no peak in the curvature data.

6. Keep the remaining possible maximum data points from this set **S**, providing that they are greater than **Smax** x a RelativeMin Input Parameter and greater than an AbsoluteMin Input Parameter.

7. If there is only one data point left, there is only one peak, and the curve has only one maximum. Define this single peak as $pk_1$. If there are two data points left, then this represents a curve with two maxima. If there are more than two peaks, take the two peaks with the highest values of the data set **S** and return the peak with the lower cycle number of these two as $pk_1$ and the peak with the higher cycle number as $pk_2$.

8. The initial condition for $\mu_1$ is then $pk_1$ and the initial condition for ($\mu_1$, $\mu_2$) is ($pk_1$, $pk_2$).

### Melting Temperature Computation

A Melting Temperature computation is illustrated in FIG. 5. In step 210, a determination is made as to whether the first derivative data set includes one or two (or three, four) peaks. If the first derivative of the melting data set is identified as having a single peak, in step 220, the maximum of the melting curve is found by a nonlinear regression (e.g., using the Levenberg-Marquardt method or other regression method) of the single component Gaussian function as described in equation (2). The initial conditions are given by the local maxima search. If the first derivative of the melting data set is identified as having two peaks (or three, four), in step 230, both local maxima of the melting curve are found by a nonlinear regression (e.g., using the Levenberg-Marquardt method) of the double component Gaussian function as described in equation (3), and the parameters $\mu_1$, $\mu_2$ (or $\mu_3$ *and* $\mu_4$) (corresponding to Tm1, Tm2, or Tm3 and Tm4) are returned. The initial conditions are given by the local maxima search. It should be appreciated that even if FIG. 5 shows a melting temperature computation for one or two peaks, the same melting temperature computation used for two peaks can also be used for three and four peaks.

In step 240, a shoulder detection process determines whether a shoulder is present for the single derivative peak situation.

If two (or three, four) peaks are found in the MELT algorithm, no further action is taken. If, instead, one peak is found, it might be possible that a shoulder is present on this peak. Details of a shoulder detection process are discussed below. If no shoulder is detected, $\mu_1$ (corresponding to Tm1) is returned. In step 250 confidence intervals are determined for the various parameters. In step 260, an $R^2$ value is calculated for the Gaussian Mixture Model used. If the $R^2$ value of the GMM1 (or GMM2, GMM3, GMM4) fit is > 0.9, or the confidence intervals for the parameters ($\mu_1$, $\sigma_1$), or ($\mu_1$, $\sigma_1$, $\mu_2$, $\sigma_2$, $\mu_3$, $\sigma_3$, $\mu_4$, $\sigma_4$ ) do not include zero, then the values of Tm1 (or Tm1, Tm2, Tm3, Tm4) are accepted in step 270. Otherwise, Tm1, (or Tm1, Tm2, Tm3, Tm4) are set as target not detected (TND) in step 280, and the optional Expert System in step 290 makes the final call of TND or Invalid.

### Shoulder Detection

A shoulder detection process is shown in FIG. 6. In step 310, a double component Gaussian model is fit to the same first derivative data using $a_{1,2} = 2$, $\mu_{1,2} = m \pm 2$, $\sigma_{1,2} = 2$ as initial conditions, where m is the melting temperature of the single peak. In step 320, the potential main peak and shoulder (lower) peak are determined as above. In step 330, a determination is made as to whether the height of the lower peak is at least a certain percentage (e.g., 0.05 or more times the higher peak) of the higher peak. If not, a shoulder not present call is returned and Tm determination processing continues as per step 250 in FIG. 5. If so, in step 340, confidence intervals are determined for the various parameters and, in step 350, an $R^2$ value is determined for the GMM2 equation. In step 360, a determination is made as to whether the confidence intervals of the means are at least a certain number of degrees Centigrade apart from each other (e.g., 3 degrees or more). If not, a shoulder not present call is returned and Tm determination processing continues as per step 250 in FIG. 5. If so, in step 370, a determination is made as to whether the confidence intervals do not contain zero or whether the $R^2$ value is greater than a threshold (e.g., 0.9). If not, a shoulder not present call is returned and Tm determination processing continues as per step 250 in FIG. 5. If so, in step 380, a determination of whether the second (2d) derivative of the double component Gaussian model (GMM2) evaluated at both $\mu_1$ and $\mu_2$ is negative. If so, a shoulder detected call is made.

Thus, assuming the LM-method converged, a shoulder is detected if ALL of the following conditions hold true:

- The first derivative data was identified to have exactly one local maximum.
- The lower peak is at least 0.05 times the higher peak in height (e.g., using "exp(-a)").
- The **confidence intervals of the means** are at least a 3 degrees Centigrade apart from each other.
- The confidence intervals of $\mu_1$, $\mu_2$, $\sigma_1$ and $\sigma_2$ do not contain zero or $R^2 > 0.9$.
- The **second derivative** of the double component Gaussian model (GMM2) evaluated at **both** u $_1$ and $\mu_2$ is negative.

In the case in which these conditions are fulfilled, a Shoulder Detection flag is raised. The above shoulder detection process is described in connection with FIG. 6 for a first derivative comprising one or two peaks. Nevertheless, as mentioned above, it should be understood that this shoulder detection process can be applied to first derivative data presenting up to four peaks.

### Expert System Checks

One or more expert system checks are implemented in step 290 (FIG. 5). In one check, it is determined whether the median of all the entire melting curve data is > 0. If this is false, then the result is reported as Invalid. In another check, it is determined whether the absolute value of the maximum of the Melt Peak fluorescent data is greater than the absolute value of the minimum of the Melt Peak fluorescent data. If this is false, then the result is reported as Invalid. In another check, it is determined whether the median of the raw fluorescence vs. temperature values from the first several (e.g., five) cycles is greater than the median of the raw fluorescence vs. temperature values from the last several (e.g., five) cycles. If this is false, then result is reported as Invalid. In yet another check, a second order polynomial is fit to the raw fluorescence vs. temperature data set. If the $R^2$ on this fit is greater than a threshold (e.g., 0.99), then no peaks are present and the result is reported as Target Not Detected (TND).

### Examples

FIGS. 7a and 7b show a raw data curve and a melting curve, respectively, in the case of a single melting peak. The calculated melting temperature is Tm = 67.8.

FIGS. 8a and 8b show a raw data curve and a melting curve, respectively, in the case of a two melting peaks. The calculated melting temperatures are Tm1 = 58.8 and Tm2 = 67.8.

FIGS. 9a and 9b show a raw data curve and a melting curve, respectively, in the case of a two melting peaks. The calculated melting temperatures are Tm1 = 57.9 and Tm2 = 68.6.

FIGS. 10a and 10b show a raw data curve and a melting curve, respectively, in the case of a single melting peak plus a shoulder. The calculated melting temperatures are Tm1 = 62.4 and Tm2 = 68.8.

FIGS. 11a and 11b show a raw data curve and a melting curve, respectively, in the case of a single melting peak plus a shoulder. The calculated melting temperatures are Tm1 = 61.3 and Tm2 = 68.1.

The Tm determination processes according to the invention can be implemented by using conventional personal computer systems including, but not limited to, an input device to input a data set, such as a keyboard, mouse, and the like; a display device to represent a specific point of interest in a region of a curve, such as a monitor; a processing device necessary to carry out each step in the method, such as a CPU; a network interface such as a modem, a data storage device to store the data set, a computer code running on the processor and the like. Furthermore, the processes may also be implemented in a PCR process according to the invention or in a PCR system according to the invention.

It should be appreciated that the Tm determination processes, including the derivative and shoulder determination processes, may be implemented in computer code running on a processor of a computer system. The code includes instructions for controlling a processor to implement various aspects and steps of the Tm determination processes. The code is typically stored on a hard disk, RAM or portable medium such as a CD, DVD, etc. Similarly, the processes may be implemented in a PCR device such as a thermocycler, or other specialized device, including a processor executing instructions stored in a memory unit coupled to the processor. Code including such instructions may be downloaded to the device memory unit over a network connection or direct connection to a code source or using a portable medium as is well known.

One skilled in the art should appreciate that the Tm determination processes of the present invention can be coded using a variety of programming languages such as C, C++, C#, Fortran, VisualBasic, etc., as well as applications such as Mathematica® which provide pre-packaged routines, functions and procedures useful for data visualization and analysis. Another example of the latter is MATLAB ®.

An example of system according to the invention is displayed in FIGS. 12 and 13. FIG. 12 shows a general block diagram explaining the relation between software and hardware resources that may be used to implement the processes and system of the invention. The system depicted on FIG. 13 comprises a kinetic PCR analysis module which may be located in a thermocycler device and an intelligence module which is part of the computer system. The data sets (PCR data sets) are transferred from the analysis module to the intelligence module or vice versa via a network connection or a direct connection. The data sets may for example be processed according to the flowcharts as depicted on FIGS. 4, 5 and 6. These flowcharts may conveniently be implemented by a software stored on the hardware of a computer system for example according to the flowchart as depicted on FIG. 12. Referring to FIG. 12, computer system (400) may comprise receiving means (410) for example for receiving fluorescence data obtained during PCR reactions, calculating means (420) for processing said data according to the processes of the invention, applying means (430) for applying the result obtained by the calculation means, and displaying means (440) for displaying the results on a computer screen. FIG. 13 illustrates the interaction between a thermocycler device and a computer system. The system comprises a kinetic PCR analysis module which may be located in a thermocycler device and an intelligence module which is part of the computer system. The data sets (PCR data sets) are transferred from the analysis module to the intelligence module or vice versa via a network connection or a direct connection. The data sets may be processed according to FIG. 12 by computer code running on the processor and being stored on the storage device of the intelligence module and after processing transferred back to the storage device of the analysis module, where the modified data may be displayed on a displaying device.

## Claims

**1.** A computer-implemented method of determining a melting temperature, Tm, of DNA, the method comprising:

- receiving a dataset representing a melt curve for a DNA sample, the dataset including a plurality of data points each having a pair of coordinate values;
- numerically determining first derivative values for data points of the melt curve;
- determining a baseline for the first derivative values;
- subtracting out the baseline from the first derivative values to produce modified first derivative values;
- determining whether the modified first derivative values include one or two or, optionally, three or four maximum values;
- if it is determined that the modified first derivative values include a single maximum value, calculating an approximation of a curve that fits the modified first derivative values by applying a Levenberg-Marquardt (LM) regression process to a first Gaussian Mixture Model function, wherein said parameters include initial conditions, wherein the single maximum value is used as an initial condition for a first parameter, and wherein the first Gaussian Mixture Model function is:

$$GMM_1 = Exp(-a_1) \cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value;
- determining whether the first derivative values include a shoulder value proximal to the first maximum value by

  - calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks;

- determining whether a shoulder value is present based on whether the height of the lower of said two peaks is at least a higher than a given percentage of the higher of said two peaks;

- outputting said first maximum value, wherein said first maximum value represents a first melting temperature Tm1 of the DNA sample; and
- if it has been detected that a shoulder value is present, outputting said shoulder value, the shoulder value representing a second melting temperature Tm2 of the DNA sample.

**2.** The method of claim 1, further including displaying the first parameter value.

**3.** The method of claim 1, wherein if it is determined that the modified first derivative values include three maximum values an approximation of a curve that fits the modified first derivative values is calculated by applying a Levenberg-Marquardt (LM) regression process to a third Gaussian Mixture Model function, wherein said parameters include initial condition, wherein the three maximum values are used as an initial conditions for the parameters, and wherein the third Gaussian Mixture Model function is:

$$GMM_3 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right)$$

wherein $\mu_1$ is the first parameter, $\mu_2$ is the second parameter, $\mu_3$ is the third parameter and wherein $a_1$, $\sigma_1$, $a_2$, $\sigma_2$, $a_3$ and $\sigma_3$ are additional parameters; wherein the value of the first parameter$\mu_1$, the second parameter$\mu_2$, and the third parameter $\mu_3$ determined by the Levenberg-Marquardt regression process represents a first, second and third maximum value; and
wherein the method further comprises outputting said first, second and third maximum values, wherein said first, second and third maximum values represent a first, second and third melting temperature Tm1, Tm2 and Tm3 of the DNA sample.

**4.** The method of claim 1, wherein if it is determined that the modified first derivative values include four maximum values an approximation of a curve that fits the modified first derivative values is calculated by applying a Levenberg-Marquardt (LM) regression process to a fourth Gaussian Mixture Model function, wherein said parameters include initial conditions, wherein the four maximum values are used as an initial condition for the parameters, and wherein the fourth Gaussian Mixture Model function is:

$$GMM_4 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right) + Exp(-a_4) \cdot Exp\left(-\frac{1}{2}\left(\frac{x-\mu_4}{\sigma_4}\right)^2\right)$$

wherein $\mu_1$ is the first parameter, $\mu_2$ is the second parameter, $\mu_3$ is the third parameter $\mu_4$ is the fourth parameter

and wherein $a_1$, $\sigma_1$, $a_2$, $\sigma_2$, $a_3$, $\sigma_3$, $a_4$ and $\sigma_4$ are additional parameters;wherein the value of the first parameter $\mu_1$, the second parameter $\mu_2$, the third parameter $\mu_3$ and the fourth parameter $\mu_4$ determined by the Levenberg-Marquardt regression process represents a first, second, third and fourth maximum value; and
wherein the method further comprises outputting said first, second, third and fourth maximum values, wherein said first, second, third and fourth maximum values represent a first, second, third and fourth melting temperature Tm1, Tm2 Tm3 and Tm4 of the DNA sample.

**5.** A computer readable medium including code for controlling a processor to determine a melting temperature, Tm, of DNA, the code including instructions to:

- receive a dataset representing a melt curve for a DNA sample, the dataset including a plurality of data points each having a pair of coordinate values;
- numerically determine first derivative values for data points of the melt curve;
- determine a baseline for the first derivative values;
- subtract out the baseline from the first derivative values to produce modified first derivative values;
- determine whether the modified first derivative values include one or two or, optionally, three or four maximum values;
- if it is determined that the modified first derivative values include a single maximum value, calculate an approximation of a curve that fits the modified first derivative values by applying a Levenberg-Marquardt (LM) regression process to a first Gaussian Mixture Model function, wherein said parameters include initial conditions, wherein the single maximum value is used as an initial condition for a first parameter, and wherein the first Gaussian Mixture Model function is:

$$GMM_1 = Exp(-a_1)\cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value;
- determine whether the first derivative values include a shoulder value proximal to the first maximum value by

- calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks;
- determining whether a shoulder value is present based on whether the height of the lower of said two peaks is at least a higher than a given percentage of the higher of said two peaks;

- output said first maximum value, wherein said first maximum value represents a first melting temperature Tm1 of the DNA sample; and
- if it has been detected that a shoulder value is present, outputting said shoulder value, the shoulder value representing a second melting temperature Tm2 of the DNA sample.

**6.** The computer readable medium of claim 5, wherein the code is adapted to perform the steps of any one of claims 2 to 4.

**7.** A kinetic Polymerase Chain Reaction (PCR) system, comprising:

- a kinetic PCR analysis module adapted to generate a melt curve dataset representing a DNA melt curve, said dataset including a plurality of data points, each having a pair of coordinate values; and
- an intelligence module adapted to process the melt curve dataset to determine a Tm value, by

- receiving a dataset representing a melt curve for a DNA sample, the dataset including a plurality of data points each having a pair of coordinate values;
- numerically determining first derivative values for data points of the melt curve;
- determining a baseline for the first derivative values;
- subtracting out the baseline from the first derivative values to produce modified first derivative values;
- determining whether the modified first derivative values include one or two or, optionally, three or four maximum values;
- if it is determined that the modified first derivative values include a single maximum value, calculating an approximation of a curve that fits the modified first derivative values by applying a Levenberg-Marquardt (LM) regression process to a first Gaussian Mixture Model function, wherein said parameters include initial conditions, wherein the single maximum value is used as an initial condition for a first parameter, and wherein the first Gaussian Mixture Model function is:

$$GMM_1 = Exp(-a_1) \cdot Exp\left(-\frac{1}{2}\left(\frac{x-\mu_1}{\sigma_1}\right)^2\right),$$

wherein $\mu_1$ is the first parameter, and wherein $a_1$ and $\sigma_1$ are additional parameters; wherein the value of the first parameter $\mu_1$ determined by the Levenberg-Marquardt regression process represents a first maximum value;
- determining whether the first derivative values include a shoulder value proximal to the first maximum value by

- calculating an approximation of a curve that fits the modified first derivative values by applying a Levenberg-Marquardt (LM) regression process to a second Gaussian Mixture Model function, wherein the second Gaussian Mixture Model function is:

$$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

wherein $\mu_1$ is a second parameter, $\mu_2$ is a third parameter and $a_1$, $\sigma_1$, $a_2$ and $\sigma_2$ are further additional parameters, wherein $a_{1,2} = 2$, $\mu_{1,2} = m \pm 2$, $\sigma_{1,2} = 2$ are used as initial conditions, where m is the first maximum value; wherein the values of the second parameter $\mu_1$ and the third parameter $\mu_2$ determined by the Levenberg-Marquardt regression process represent two peaks;
- determining whether a shoulder value is present based on whether the height of the lower of said two peaks is at least a higher than a given percentage of the higher of said two peaks;

- outputting said first maximum value, wherein said first maximum value represents a first melting temperature Tm1 of the DNA sample; and
- if it has been detected that a shoulder value is present, outputting said shoulder value, the shoulder value representing a second melting temperature Tm2 of the DNA sample.

**8.** The system of claim 7, wherein the kinetic PCR analysis module is resident in a kinetic thermocycler device, and wherein the intelligence module includes a processor communicably coupled to the analysis module or wherein the intelligence module includes a processor resident in a computer system coupled to the analysis module by one of a network connection or a direct connection.

**9.** The system of claim 7, further including a display module, wherein outputting includes displaying the Tm value on the display module.

**10.** The system of claim 7, wherein the system is adapted to perform the steps of any one of claims 2 to 4.

**Patentansprüche**

1. Computerimplementiertes Verfahren zum Bestimmen einer Schmelztemperatur, Tm, von DNA, wobei das Verfahren Folgendes umfasst:

   - Empfangen eines Datensatzes, der eine Schmelzkurve für eine DNA-Probe darstellt, wobei der Datensatz eine Vielzahl von Datenpunkten einschließt, die jeweils ein Paar von Koordinatenwerten aufweisen;
   - numerisches Bestimmen von Werten erster Ableitung für Datenpunkte der Schmelzkurve;
   - Bestimmen einer Grundlinie für die Werte erster Ableitung;
   - Subtrahieren der Grundlinie von den Werten erster Ableitung zur Erzeugung modifizierter Werte erster Ableitung;
   - Bestimmen, ob die modifizierten Werte erster Ableitung einen oder zwei oder gegebenenfalls drei oder vier Höchstwert(e) einschließen;
   - wenn bestimmt wird, dass die modifizierten Werte erster Ableitung nur einen Höchstwert einschließen, Berechnen einer Näherung einer Kurve, die den modifizierten Werten erster Ableitung entspricht, durch Anwenden eines Levenberg-Marquardt-(LM)-Regressionsprozesses auf eine erste Gaußsche Mischmodell-Funktion, wobei die Parameter Ausgangsbedingungen einschließen, wobei der eine Höchstwert als eine Ausgangsbedingung für einen ersten Parameter verwendet wird und wobei die erste Gaußsche Mischmodell-Funktion Folgende ist:

   $$GMM_1 = Exp(-a_1) \cdot Exp\left(-\frac{1}{2}\left(\frac{x-\mu_1}{\sigma_1}\right)^2\right),$$

   wobei $\mu_1$ der erste Parameter ist und wobei $a_1$ und $\sigma_1$ zusätzliche Parameter sind; wobei der Wert des ersten Parameters $\mu_1$, bestimmt mittels des Levenberg-Marquardt-Regressionsprozesses, einen ersten Höchstwert darstellt;
   - Bestimmen, ob die Werte erster Ableitung einen zu dem ersten Höchstwert proximalen Schulterwert einschließen, durch

     - Berechnen einer Näherung einer Kurve, die den modifizierten Werten erster Ableitung entspricht, durch Anwenden eines Levenberg-Marquardt-(LM)-Regressionsprozesses auf eine zweite Gaußsche Mischmodell-Funktion, wobei die zweite Gaußsche Mischmodell-Funktion Folgende ist:

     $$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right),$$

     wobei $\mu_1$ ein zweiter Parameter ist, $\mu_2$ ein dritter Parameter ist und $a_1$, $\sigma_1$, $a_2$ und $\sigma_2$ weitere zusätzliche Parameter sind, wobei $a_{1,2} = 2$, $\mu_{1,2} = m \pm 2$, $\sigma_{1,2} = 2$ als Ausgangsbedingungen verwendet werden, wobei m der erste Höchstwert ist; wobei die Werte des zweiten Parameters $\mu_1$ und des dritten Parameters $\mu_2$, bestimmt mittels des Levenberg-Marquardt-Regressionsprozesses, zwei Peaks darstellen;
     - Bestimmen, ob ein Schulterwert vorliegt, basierend darauf, ob die Höhe des niedrigsten der beiden Peaks mindestens höher ist als ein vorgegebener Prozentsatz des höheren der beiden Peaks;

   - Ausgeben des ersten Höchstwertes, wobei der erste Höchstwert eine erste Schmelztemperatur Tm1 der DNA-Probe darstellt; und
   - wenn nachgewiesen wurde, dass ein Schulterwert vorliegt, Ausgeben des Schulterwertes, wobei der Schulterwert eine zweite Schmelztemperatur Tm2 der DNA-Probe darstellt.

2. Verfahren nach Anspruch 1, das ferner das Anzeigen des ersten Parameterwertes einschließt.

3. Verfahren nach Anspruch 1, wobei, wenn bestimmt wird, dass die modifizierten Werte erster Ableitung drei Höchstwerte einschließen, eine Näherung einer Kurve, die den modifizierten Werten erster Ableitung entspricht, durch Anwenden eines Levenberg-Marquardt-(LM)-Regressionsprozesses auf eine dritte Gaußsche Mischmodell-Funktion berechnet wird, wobei die Parameter Ausgangsbedingungen einschließen, wobei die drei Höchstwerte als eine Ausgangsbedingung für die Parameter verwendet werden und wobei die dritte Gaußsche Mischmodell-Funktion Folgende ist:

$$GMM_3 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) + Exp(-a_3)\cdot Exp\left(-\frac{1}{2}\left(\frac{x-\mu_3}{\sigma_3}\right)^2\right),$$

wobei $\mu_1$ der erste Parameter ist, $\mu_2$ der zweite Parameter ist, $\mu_3$ der dritte Parameter ist und wobei $a_1$, $\sigma_1$, $a_2$, $\sigma_2$, $a_3$ und $\sigma_3$ zusätzliche Parameter sind; wobei der Wert des ersten Parameters $\mu_1$, des zweiten Parameters $\mu_2$ und des dritten Parameters $\mu_3$, bestimmt mittels des Levenberg-Marquardt-Regressionsprozesses, einen ersten, zweiten und dritten Höchstwert darstellen; und

wobei das Verfahren ferner das Ausgeben des ersten, zweiten und dritten Höchstwertes umfasst, wobei der erste, zweite und dritte Höchstwert eine erste, zweite und dritte Schmelztemperatur Tm1, Tm2 und Tm3 der DNA-Probe darstellen.

**4.** Verfahren nach Anspruch 1, wobei, wenn bestimmt wird, dass die modifizierten Werte erster Ableitung vier Höchstwerte einschließen, eine Näherung einer Kurve, die den modifizierten Werten erster Ableitung entspricht, durch Anwenden eines Levenberg-Marquardt-(LM)-Regressionsprozesses auf eine vierte Gaußsche Mischmodell-Funktion berechnet wird, wobei die Parameter Ausgangsbedingungen einschließen, wobei die vier Höchstwerte als eine Ausgangsbedingung für die Parameter verwendet werden und wobei die vierte Gaußsche Mischmodell-Funktion Folgende ist:

$$GMM_4 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right) +$$
$$Exp(-a_3)\cdot Exp\left(-\frac{1}{2}\left(\frac{x-\mu_3}{\sigma_3}\right)^2\right) + Exp(-a_4)\cdot Exp\left(-\frac{1}{2}\left(\frac{x-\mu_4}{\sigma_4}\right)^2\right),$$

wobei $\mu_1$ der erste Parameter ist, $\mu_2$ der zweite Parameter ist, $\mu_3$ der dritte Parameter ist, $\mu_4$ der vierte Parameter ist und wobei $a_1$, $\sigma_1$, $a_2$, $\sigma_2$, $a_3$, $\sigma_3$, $a_4$ und $\sigma_4$ zusätzliche Parameter sind; wobei der Wert des ersten Parameters $\mu_1$, des zweiten Parameters $\mu_2$, des dritten Parameters $\mu_3$ und des vierten Parameters $\mu_4$, bestimmt mittels des Levenberg-Marquardt-Regressionsprozesses, einen ersten, zweiten, dritten und vierten Höchstwert darstellen; und

wobei das Verfahren ferner das Ausgeben des ersten, zweiten, dritten und vierten Höchstwertes umfasst, wobei der erste, zweite, dritte und vierte Höchstwert eine erste, zweite, dritte und vierte Schmelztemperatur Tm1, Tm2, Tm3 und Tm4 der DNA-Probe darstellen.

**5.** Computerlesbares Medium, das einen Code zum Steuern eines Prozessors zum Bestimmen einer Schmelztemperatur, Tm, von DNA einschließt, wobei der Code die folgenden Anweisungen einschließt:

- Empfangen eines Datensatzes, der eine Schmelzkurve für eine DNA-Probe darstellt, wobei der Datensatz eine Vielzahl von Datenpunkten einschließt, die jeweils ein Paar von Koordinatenwerten aufweisen;
- numerisches Bestimmen von Werten erster Ableitung für Datenpunkte der Schmelzkurve;
- Bestimmen einer Grundlinie für die Werte erster Ableitung;
- Subtrahieren der Grundlinie von den Werten erster Ableitung zur Erzeugung modifizierter Werte erster Ableitung;
- Bestimmen, ob die modifizierten Werte erster Ableitung einen oder zwei oder gegebenenfalls drei oder vier Höchstwerte einschließen;
- wenn bestimmt wird, dass die modifizierten Werte erster Ableitung nur einen Höchstwert einschließen, Berechnen einer Näherung einer Kurve, die den modifizierten Werten erster Ableitung entspricht, durch Anwenden eines Levenberg-Marquardt-(LM)-Regressionsprozesses auf eine erste Gaußsche Mischmodell-Funktion, wobei die Parameter Ausgangsbedingungen einschließen, wobei der eine Höchstwert als eine Ausgangsbedingung für einen ersten Parameter verwendet wird und wobei die erste Gaußsche Mischmodell-Funktion Folgende ist:

$$GMM_1 = Exp(-a_1)\cdot Exp\left(-\frac{1}{2}\left(\frac{x-\mu_1}{\sigma_1}\right)^2\right),$$

wobei $\mu_1$ der erste Parameter ist und wobei $a_1$ und $\sigma_1$ zusätzliche Parameter sind; wobei der Wert des ersten Parameters $\mu_1$, bestimmt mittels des Levenberg-Marquardt-Regressionsprozesses, einen ersten Höchstwert darstellt;
- Bestimmen, ob die Werte erster Ableitung einen zu dem ersten Höchstwert proximalen Schulterwert einschließen, durch

- Berechnen einer Näherung einer Kurve, die den modifizierten Werten erster Ableitung entspricht, durch Anwenden eines Levenberg-Marquardt-(LM)-Regressionsprozesses auf eine zweite Gaußsche Mischmodell-Funktion, wobei die zweite Gaußsche Mischmodell-Funktion Folgende ist:

$$GMM_2 = Exp(-a_1)\cdot Exp\left(-\frac{1}{2}\left(\frac{x-\mu_1}{\sigma_1}\right)^2\right) + Exp(-a_2)\cdot Exp\left(-\frac{1}{2}\left(\frac{x-\mu_2}{\sigma_2}\right)^2\right),$$

wobei $\mu_1$ ein zweiter Parameter ist, $\mu_2$ ein dritter Parameter ist und $a_1$, $\sigma_1$, $a_2$ und $\sigma_2$ weitere zusätzliche Parameter sind, wobei $a_{1,2} = 2$, $\mu_{1,2} = m \pm 2$, $\sigma_{1,2} = 2$ als Ausgangsbedingungen verwendet werden, wobei m der erste Höchstwert ist; wobei die Werte des zweiten Parameters $\mu_1$ und des dritten Parameters $\mu_2$, bestimmt mittels des Levenberg-Marquardt-Regressionsprozesses, zwei Peaks darstellen;
- Bestimmen, ob ein Schulterwert vorliegt, basierend darauf, ob die Höhe des niedrigsten der beiden Peaks mindestens höher ist als ein vorgegebener Prozentsatz des höheren der beiden Peaks;

- Ausgeben des ersten Höchstwertes, wobei der erste Höchstwert eine erste Schmelztemperatur Tm1 der DNA-Probe darstellt; und
- wenn nachgewiesen wurde, dass ein Schulterwert vorliegt, Ausgeben des Schulterwertes, wobei der Schulterwert eine zweite Schmelztemperatur Tm2 der DNA-Probe darstellt.

**6.** Computerlesbares Medium nach Anspruch 5, wobei der Code dafür ausgelegt ist, die Schritte nach einem der Ansprüche 2 bis 4 auszuführen.

**7.** Kinetisches Polymerasekettenreaktion-(PCR)-System, umfassend:

- ein kinetisches PCR-Analysemodul, das dafür ausgelegt ist, einen Schmelzkurvendatensatz, der eine DNA-Schmelzkurve darstellt, zu erzeugen, wobei der Datensatz eine Vielzahl von Datenpunkten einschließt, die jeweils ein Paar von Koordinatenwerten aufweisen; und
- ein Intelligenzmodul, das dafür ausgelegt ist, den Schmelzkurvendatensatz zu verarbeiten, um einen Tm-Wert zu bestimmen durch
- Empfangen eines Datensatzes, der eine Schmelzkurve für eine DNA-Probe darstellt, wobei der Datensatz eine Vielzahl von Datenpunkten einschließt, die jeweils ein Paar von Koordinatenwerten aufweisen;
- numerisches Bestimmen von Werten erster Ableitung für Datenpunkte der Schmelzkurve;
- Bestimmen einer Grundlinie für die Werte erster Ableitung;
- Subtrahieren der Grundlinie von den Werten erster Ableitung zur Erzeugung modifizierter Werte erster Ableitung;
- Bestimmen, ob die modifizierten Werte erster Ableitung einen oder zwei oder gegebenenfalls drei oder vier Höchstwerte einschließen;
- wenn bestimmt wird, dass die modifizierten Werte erster Ableitung nur einen Höchstwert einschließen, Berechnen einer Näherung einer Kurve, die den modifizierten Werten erster Ableitung entspricht, durch Anwenden eines Levenberg-Marquardt-(LM)-Regressionsprozesses auf eine erste Gaußsche Mischmodell-Funktion, wobei die Parameter Ausgangsbedingungen einschließen, wobei der eine Höchstwert als eine Ausgangsbedingung für einen ersten Parameter verwendet wird und wobei die erste Gaußsche Mischmodell-Funktion Folgende ist:

$$GMM_1 = Exp(-a_1)\cdot Exp\left(-\frac{1}{2}\left(\frac{x-\mu_1}{\sigma_1}\right)^2\right),$$

wobei $\mu_1$ der erste Parameter ist und wobei $a_1$ und $\sigma_1$ zusätzliche Parameter sind; wobei der Wert des ersten Parameters $\mu_1$, bestimmt mittels des Levenberg-Marquardt-Regressionsprozesses, einen ersten Höchstwert darstellt;
- Bestimmen, ob die Werte erster Ableitung einen zu dem ersten Höchstwert proximalen Schulterwert einschließen, durch

- Berechnen einer Näherung einer Kurve, die den modifizierten Werten erster Ableitung entspricht, durch Anwenden eines Levenberg-Marquardt-(LM)-Regressionsprozesses auf eine zweite Gaußsche Mischmodell-Funktion, wobei die zweite Gaußsche Mischmodell-Funktion Folgende ist:

$$GMM_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right),$$

wobei $\mu_1$ ein zweiter Parameter ist, $\mu_2$ ein dritter Parameter ist und $a_1$, $\sigma_1$, $a_2$ und $\sigma_2$ weitere zusätzliche Parameter sind, wobei $a_{1,2} = 2$, $\mu_{1,2} = m \pm 2$, $\sigma_{1,2} = 2$ als Ausgangsbedingungen verwendet werden, wobei m der erste Höchstwert ist; wobei die Werte des zweiten Parameters $\mu_1$ und des dritten Parameters $\mu_2$, bestimmt mittels des Levenberg-Marquardt-Regressionsprozesses, zwei Peaks darstellen;
- Bestimmen, ob ein Schulterwert vorliegt, basierend darauf, ob die Höhe des niedrigsten der beiden Peaks mindestens höher ist als ein vorgegebener Prozentsatz des höheren der beiden Peaks;

- Ausgeben des ersten Höchstwertes, wobei der erste Höchstwert eine erste Schmelztemperatur Tm1 der DNA-Probe darstellt; und
- wenn nachgewiesen wurde, dass ein Schulterwert vorliegt, Ausgeben des Schulterwertes, wobei der Schulterwert eine zweite Schmelztemperatur Tm2 der DNA-Probe darstellt.

**8.** System nach Anspruch 7, wobei das kinetische PCR-Analysemodul in einer kinetischen Thermocycler-Vorrichtung untergebracht ist und wobei das Intelligenzmodul einen Prozessor einschließt, der kommunikationsfähig an das Analysemodul gekoppelt ist, oder wobei das Intelligenzmodul einen Prozessor einschließt, der in einem Computersystem untergebracht ist, das durch eine von einer Netzwerkverbindung oder einer direkten Verbindung an das Analysemodul gekoppelt ist.

**9.** System nach Anspruch 7, das ferner ein Anzeigemodul einschließt, wobei das Ausgeben das Anzeigen des Tm-Wertes auf dem Anzeigemodul einschließt.

**10.** System nach Anspruch 7, wobei das System dafür ausgelegt ist, die Schritte nach einem der Ansprüche 2 bis 4 auszuführen.

## Revendications

**1.** Procédé mis en oeuvre par ordinateur de détermination d'une température de fusion, Tf, de l'ADN, le procédé comprenant :

- la réception d'un jeu de données représentant une courbe de fusion pour un échantillon d'ADN, le jeu de données incluant une pluralité de points de données ayant chacun une paire de valeurs de coordonnées ;
- la détermination numérique de valeurs de dérivée première pour les points de données de la courbe de fusion ;
- la détermination d'une ligne de base pour les valeurs de dérivée première ;
- la soustraction de la ligne de base des valeurs de dérivée première pour produire des valeurs de dérivée première modifiées ;
- la détermination si les valeurs de dérivée première modifiées incluent une ou deux ou, éventuellement, trois ou quatre valeurs maximales ;
- s'il est déterminé que les valeurs de dérivée première modifiées incluent une valeur maximale unique, le calcul d'une approximation d'une courbe qui s'ajuste aux valeurs de dérivée première modifiées par application d'un procédé de régression de Levenberg-Marquardt (LM) à une première fonction de modèle de mélange gaussien, dans lequel lesdits paramètres incluent des conditions initiales, dans lequel la valeur maximale unique est utilisée en tant que condition initiale pour un premier paramètre, et dans lequel la première fonction de modèle de mélange gaussien est :

$$MMG_1 = Exp(-a_1) \cdot Exp\left(-\frac{1}{2}\left(\frac{x-\mu_1}{\sigma_1}\right)^2\right),$$

dans lequel $\mu_1$ est le premier paramètre, et dans lequel $a_1$ et $\sigma_1$ sont des paramètres supplémentaires ; dans lequel la valeur du premier paramètre $\mu_1$ déterminée par le procédé de régression de Levenberg-Marquardt représente une première valeur maximale ;
- la détermination si les valeurs de dérivée première incluent une valeur d'épaulement proximale de la première valeur maximale par

- le calcul d'une approximation d'une courbe qui s'ajuste aux valeurs de dérivée première modifiées par application d'un procédé de régression de Levenberg-Marquardt (LM) à une deuxième fonction de modèle de mélange gaussien, dans lequel la deuxième fonction de modèle de mélange gaussien est :

$$MMG_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

dans lequel $\mu_1$ est un deuxième paramètre, $\mu_2$ est un troisième paramètre et $a_1$, $\sigma_1$, $a_2$ et $\sigma_2$ sont d'autres paramètres supplémentaires, dans lequel $a_{1,2} = 2$, $\mu_{1,2} = m \pm 2$, $\sigma_{1,2} = 2$ sont utilisés en tant que conditions initiales, où m est la première valeur maximale ; dans lequel les valeurs du deuxième paramètre $\mu_1$ et du troisième paramètre $\mu_2$ déterminées par le procédé de régression de Levenberg-Marquardt représentent deux pics ;
- la détermination si une valeur d'épaulement est présente sur la base de si la hauteur du plus bas desdits deux pics est au moins plus élevée qu'un pourcentage donné du plus élevé desdits deux pics ;

- la production de ladite première valeur maximale, dans lequel ladite première valeur maximale représente une première température de fusion Tf1 de l'échantillon d'ADN ; et
- s'il a été détecté qu'une valeur d'épaulement est présente, la production de ladite valeur d'épaulement, la valeur d'épaulement représentant une deuxième température de fusion Tf2 de l'échantillon d'ADN.

**2.** Procédé selon la revendication 1, incluant en outre l'affichage de la première valeur de paramètre.

**3.** Procédé selon la revendication 1, dans lequel s'il est déterminé que les valeurs de dérivée première modifiées incluent trois valeurs maximales, une approximation d'une courbe qui s'ajuste aux valeurs de dérivée première modifiées est calculée par application d'un procédé de régression de Levenberg-Marquardt (LM) à une troisième fonction de modèle de mélange gaussien, dans lequel lesdits paramètres incluent des conditions initiales, dans lequel les trois valeurs maximales sont utilisées en tant que conditions initiales pour les paramètres, et dans lequel la troisième fonction de modèle de mélange gaussien est :

$$MMG_3 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right) + Exp(-a_3) \cdot Exp\left(-\frac{1}{2}\left(\frac{x-\mu_3}{\sigma_3}\right)^2\right)$$

dans lequel $\mu_1$ est le premier paramètre, $\mu_2$ est le deuxième paramètre, $\mu_3$ est le troisième paramètre et dans lequel $a_1$, $\sigma_1$, $a_2$, $\sigma_2$, $a_3$ et $\sigma_3$ sont des paramètres supplémentaires ; dans lequel les valeurs du premier paramètre $\mu_1$, du deuxième paramètre $\mu_2$ et du troisième paramètre $\mu_3$ déterminées par le procédé de régression de Levenberg-Marquardt représentent une première, deuxième et troisième valeur maximale ; et
dans lequel le procédé comprend en outre la production desdites première, deuxième et troisième valeurs maximales, dans lequel lesdites première, deuxième et troisième valeurs maximales représentent une première, deuxième et troisième température de fusion Tf1, Tf2 et Tf3 de l'échantillon d'ADN.

**4.** Procédé selon la revendication 1, dans lequel s'il est déterminé que les valeurs de dérivée première modifiées incluent quatre valeurs maximales, une approximation d'une courbe qui s'ajuste aux valeurs de dérivée première modifiées est calculée par application d'un procédé de régression de Levenberg-Marquardt (LM) à une quatrième fonction de modèle de mélange gaussien, dans lequel lesdits paramètres incluent des conditions initiales, dans lequel les quatre valeurs maximales sont utilisées en tant que condition initiale pour les paramètres, et dans lequel la quatrième fonction de modèle de mélange gaussien est :

$$MMG_4 = Exp(-a_1) \cdot Exp\left(-\frac{1}{2}\left(\frac{x - \mu_1}{\sigma_1}\right)^2\right) + Exp(-a_2) \cdot Exp\left(-\frac{1}{2}\left(\frac{x - \mu_2}{\sigma_2}\right)^2\right)$$

$$+ Exp(-a_3) \cdot Exp\left(-\frac{1}{2}\left(\frac{x - \mu_3}{\sigma_3}\right)^2\right) + Exp(-a_4) \cdot Exp\left(-\frac{1}{2}\left(\frac{x - \mu_4}{\sigma_4}\right)^2\right)$$

dans lequel $\mu_1$ est le premier paramètre, $\mu_2$ est le deuxième paramètre, $\mu_3$ est le troisième paramètre, $\mu_4$ est le quatrième paramètre et dans lequel $a_1$, $\sigma_1$, $a_2$, $\sigma_2$, $a_3$, $\sigma_3$, $a_4$ et $\sigma_4$ sont des paramètres supplémentaires ; dans lequel les valeurs du premier paramètre $\mu_1$, du deuxième paramètre $\mu_2$, du troisième paramètre $\mu_3$ et du quatrième paramètre $\mu_4$ déterminées par le procédé de régression de Levenberg-Marquardt représentent une première, deuxième, troisième et quatrième valeur maximale ; et
dans lequel le procédé comprend en outre la production desdites première, deuxième, troisième et quatrième valeurs maximales, dans lequel lesdites première, deuxième, troisième et quatrième valeurs maximales représentent une première, deuxième, troisième et quatrième température de fusion Tf1, Tf2, Tf3 et Tf4 de l'échantillon d'ADN.

**5.** Support lisible par ordinateur incluant un code pour commander un processeur afin de déterminer une température de fusion, Tf, de l'ADN, le code incluant des instructions pour :

- recevoir un jeu de données représentant une courbe de fusion pour un échantillon d'ADN, le jeu de données incluant une pluralité de points de données ayant chacun une paire de valeurs de coordonnées ;
- déterminer numériquement les valeurs de dérivée première pour les points de données de la courbe de fusion ;
- déterminer une ligne de base pour les valeurs de dérivée première ;
- soustraire la ligne de base des valeurs de dérivée première pour produire des valeurs de dérivée première modifiées ;
- déterminer si les valeurs de dérivée première modifiée incluent une ou deux ou, éventuellement, trois ou quatre valeurs maximales ;
- s'il est déterminé que les valeurs de dérivée première modifiées incluent une valeur maximale unique, calculer une approximation d'une courbe qui s'ajuste aux valeurs de dérivée première modifiées par application d'un procédé de régression de Levenberg-Marquardt (LM) à une première fonction de modèle de mélange gaussien, dans lequel lesdits paramètres incluent des conditions initiales, dans lequel la valeur maximale unique est utilisée en tant que condition initiale pour un premier paramètre, et dans lequel la première fonction de modèle de mélange gaussien est :

$$MMG_1 = Exp(-a_1) \cdot Exp\left(-\frac{1}{2}\left(\frac{x - \mu_1}{\sigma_1}\right)^2\right),$$

dans lequel $\mu_1$ est le premier paramètre, et dans lequel $a_1$ et $\sigma_1$ sont des paramètres supplémentaires ; dans lequel la valeur du premier paramètre $\mu_1$ déterminée par le procédé de régression de Levenberg-Marquardt représente une première valeur maximale ;
- déterminer si les valeurs de dérivée première incluent une valeur d'épaulement proximale de la première valeur maximale par

- le calcul d'une approximation d'une courbe qui s'ajuste aux valeurs de dérivée première modifiées par application d'un procédé de régression de Levenberg-Marquardt (LM) à une deuxième fonction de modèle de mélange gaussien, dans lequel la deuxième fonction de modèle de mélange gaussien est :

$$MMG_2 = Exp(-a_1) \cdot Exp\left(-\frac{1}{2}\left(\frac{x - \mu_1}{\sigma_1}\right)^2\right) + Exp(-a_2) \cdot Exp\left(-\frac{1}{2}\left(\frac{x - \mu_2}{\sigma_2}\right)^2\right)$$

dans lequel $\mu_1$ est un deuxième paramètre, $\mu_2$ est un troisième paramètre et $a_1$, $\sigma_1$, $a_2$ et $\sigma_2$ sont d'autres paramètres supplémentaires, dans lequel $a_{1,2} = 2$, $\mu_{1,2} = m \pm 2$, $\sigma_{1,2} = 2$ sont utilisés en tant que conditions initiales, où m est la première valeur maximale ; dans lequel les valeurs du deuxième paramètre $\mu_1$ et du troisième paramètre $\mu_2$ déterminées par le procédé de régression de Levenberg-Marquardt représentent deux pics ;
- la détermination si une valeur d'épaulement est présente sur la base de si la hauteur du plus bas desdits deux pics est au moins plus élevée qu'un pourcentage donné du plus élevé desdits deux pics ;

- produire ladite première valeur maximale, dans lequel ladite première valeur maximale représente une première température de fusion Tf1 de l'échantillon d'ADN ; et
- s'il a été détecté qu'une valeur d'épaulement est présente, la production de ladite valeur d'épaulement, la valeur d'épaulement représentant une deuxième température de fusion Tf2 de l'échantillon d'ADN.

**6.** Support lisible par ordinateur selon la revendication 5, dans lequel le code est adapté pour réaliser les étapes selon l'une quelconque des revendications 2 à 4.

**7.** Système de réaction en chaîne par polymérase (PCR) cinétique, comprenant :

- un module d'analyse par PCR cinétique adapté pour générer un jeu de données de courbe de fusion représentant une courbe de fusion d'ADN, ledit jeu de données incluant une pluralité de points de données, chacun ayant une paire de valeurs de coordonnées ; et
- un module d'intelligence adapté pour traiter le jeu de données de courbe de fusion afin de déterminer une valeur Tf, par

- la réception d'un jeu de données représentant une courbe de fusion pour un échantillon d'ADN, le jeu de données incluant une pluralité de points de données ayant chacun une paire de valeurs de coordonnées ;
- la détermination numérique de valeurs de dérivée première pour les points de données de la courbe de fusion ;
- la détermination d'une ligne de base pour les valeurs de dérivée première ;
- la soustraction de la ligne de base des valeurs de dérivée première pour produire des valeurs de dérivée première modifiées ;
- la détermination si les valeurs de dérivée première modifiées incluent une ou deux ou, éventuellement, trois ou quatre valeurs maximales ;
- s'il est déterminé que les valeurs de dérivée première modifiées incluent une valeur maximale unique, le calcul d'une approximation d'une courbe qui s'ajuste aux valeurs de dérivée première modifiées par application d'un procédé de régression de Levenberg-Marquardt (LM) à une première fonction de modèle de mélange gaussien, dans lequel lesdits paramètres incluent des conditions initiales, dans lequel la valeur maximale unique est utilisée en tant que condition initiale pour un premier paramètre, et dans lequel la première fonction de modèle de mélange gaussien est :

$$MMG_1 = Exp(-a_1) \cdot Exp\left(-\frac{1}{2}\left(\frac{x-\mu_1}{\sigma_1}\right)^2\right),$$

dans lequel $\mu_1$ est le premier paramètre, et dans lequel $a_1$ et $\sigma_1$ sont des paramètres supplémentaires ; dans lequel la valeur du premier paramètre $\mu_1$ déterminée par le procédé de régression de Levenberg-Marquardt représente une première valeur maximale ;
- la détermination si les valeurs de dérivée première incluent une valeur d'épaulement proximale de la première valeur maximale par

- le calcul d'une approximation d'une courbe qui s'ajuste aux valeurs de dérivée première modifiées par application d'un procédé de régression de Levenberg-Marquardt (LM) à une deuxième fonction de modèle de mélange gaussien, dans lequel la deuxième fonction de modèle de mélange gaussien est :

$$MMG_2 = Exp(-a_1) \cdot Exp\left(-\frac{1}{2}\left(\frac{x-\mu_1}{\sigma_1}\right)^2\right) + Exp(-a_2) \cdot Exp\left(-\frac{1}{2}\left(\frac{x-\mu_2}{\sigma_2}\right)^2\right)$$

dans lequel $\mu_1$ est un deuxième paramètre, $\mu_2$ est un troisième paramètre et $a_1$, $\sigma_1$, $a_2$ et $\sigma_2$ sont d'autres paramètres supplémentaires, dans lequel $a_{1,2} = 2$, $\mu_{1,2} = m \pm 2$, $\sigma_{1,2} = 2$ sont utilisés en tant que conditions initiales, où m est la première valeur maximale ; dans lequel les valeurs du deuxième paramètre $\mu_1$ et du troisième paramètre $\mu_2$ déterminées par le procédé de régression de Levenberg-Marquardt représentent deux pics ;
- la détermination si une valeur d'épaulement est présente sur la base de si la hauteur du plus bas desdits deux pics est au moins plus élevée qu'un pourcentage donné du plus élevé desdits deux pics ;

- la production de ladite première valeur maximale, dans lequel ladite première valeur maximale représente une première température de fusion Tf1 de l'échantillon d'ADN ; et
- s'il a été détecté qu'une valeur d'épaulement est présente, la production de ladite valeur d'épaulement, la valeur d'épaulement représentant une deuxième température de fusion Tf2 de l'échantillon d'ADN.

**8.** Système selon la revendication 7, dans lequel le module d'analyse par PCR cinétique réside dans un dispositif thermocycleur cinétique, et dans lequel le module d'intelligence inclut un processeur couplé de manière communicante au module d'analyse ou dans lequel le module d'intelligence inclut un processeur résidant dans un système informatique couplé au module d'analyse par une parmi une connexion réseau ou une connexion directe.

**9.** Système selon la revendication 7, incluant en outre un module d'affichage, dans lequel la production inclut l'affichage de la valeur Tf sur le module d'affichage.

**10.** Système selon la revendication 7, dans lequel le système est adapté pour réaliser les étapes selon l'une quelconque des revendications 2 à 4.

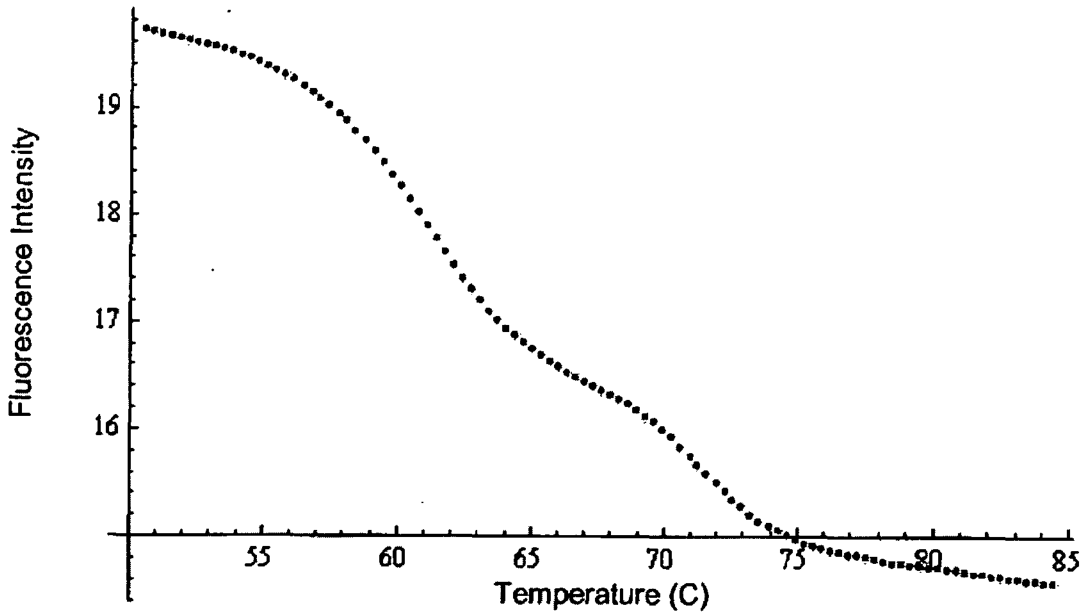


**Figure 1: Fluorescence Intensity vs. Temperature**

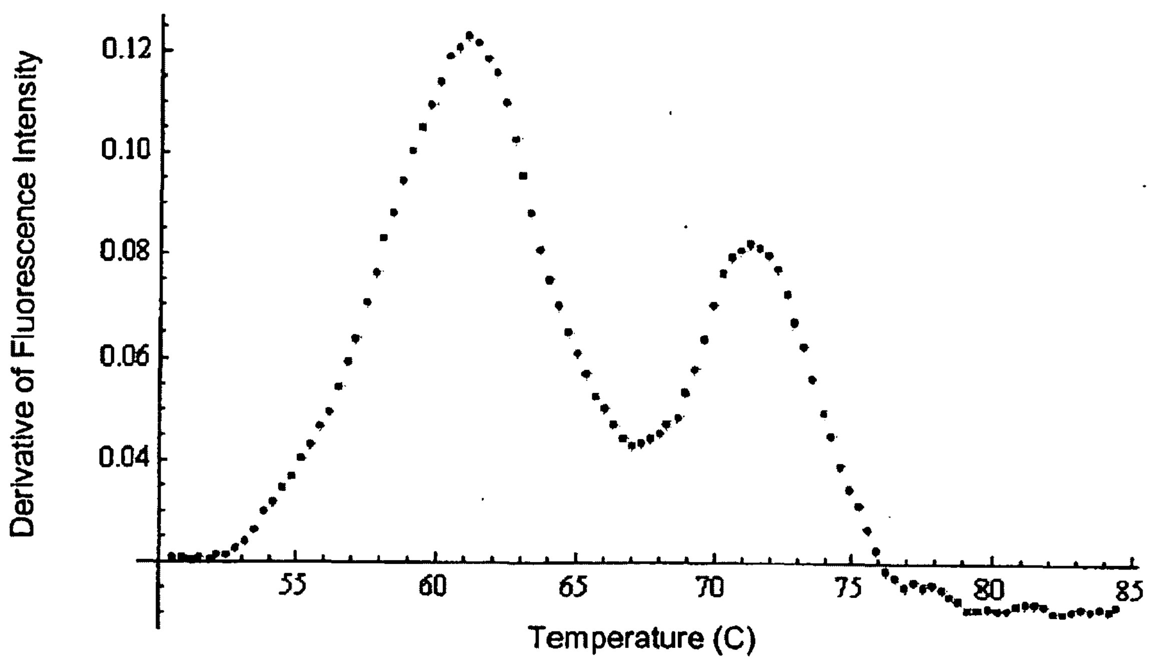


**Figure 2: First Derivative of Fluorescence Intensity vs. Temperature**

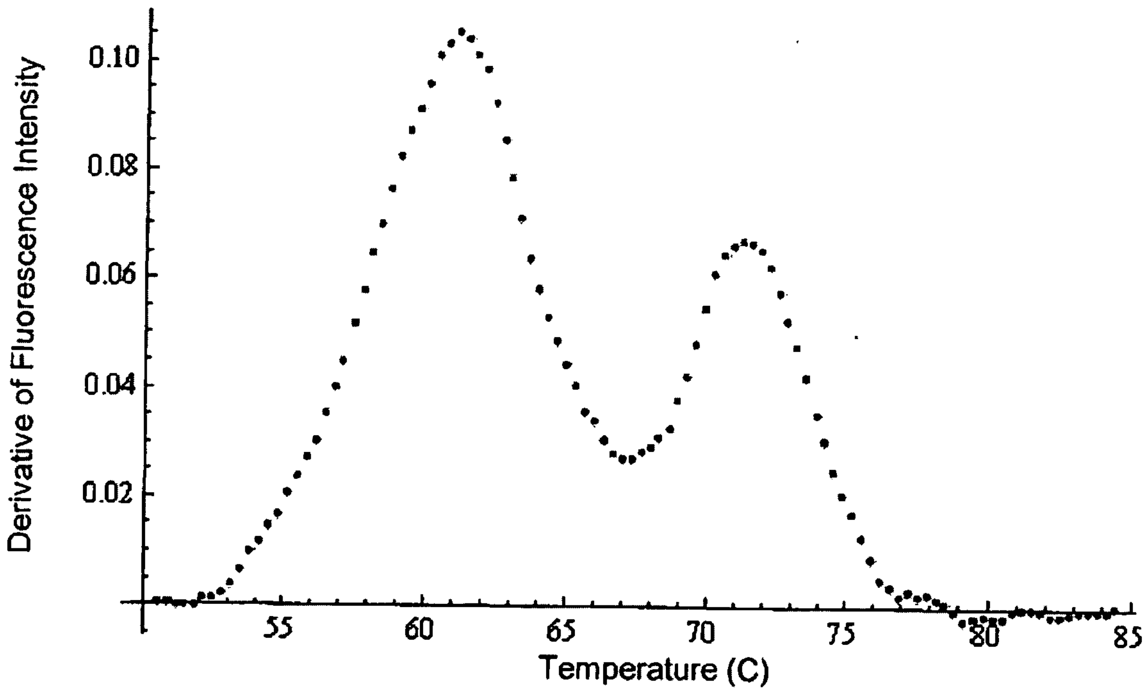


**Figure 3: Baseline Subtracted First Derivative of Fluorescence Intensity vs. Temperature**

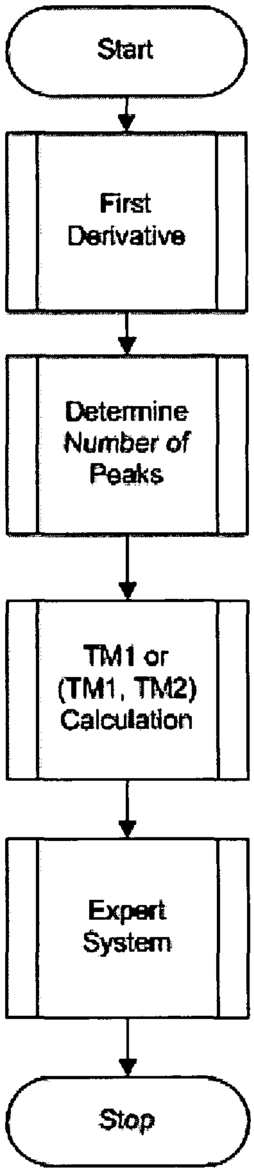


**Figure 4: Major Processing Steps of the Equation-Less Melting Algorithm**

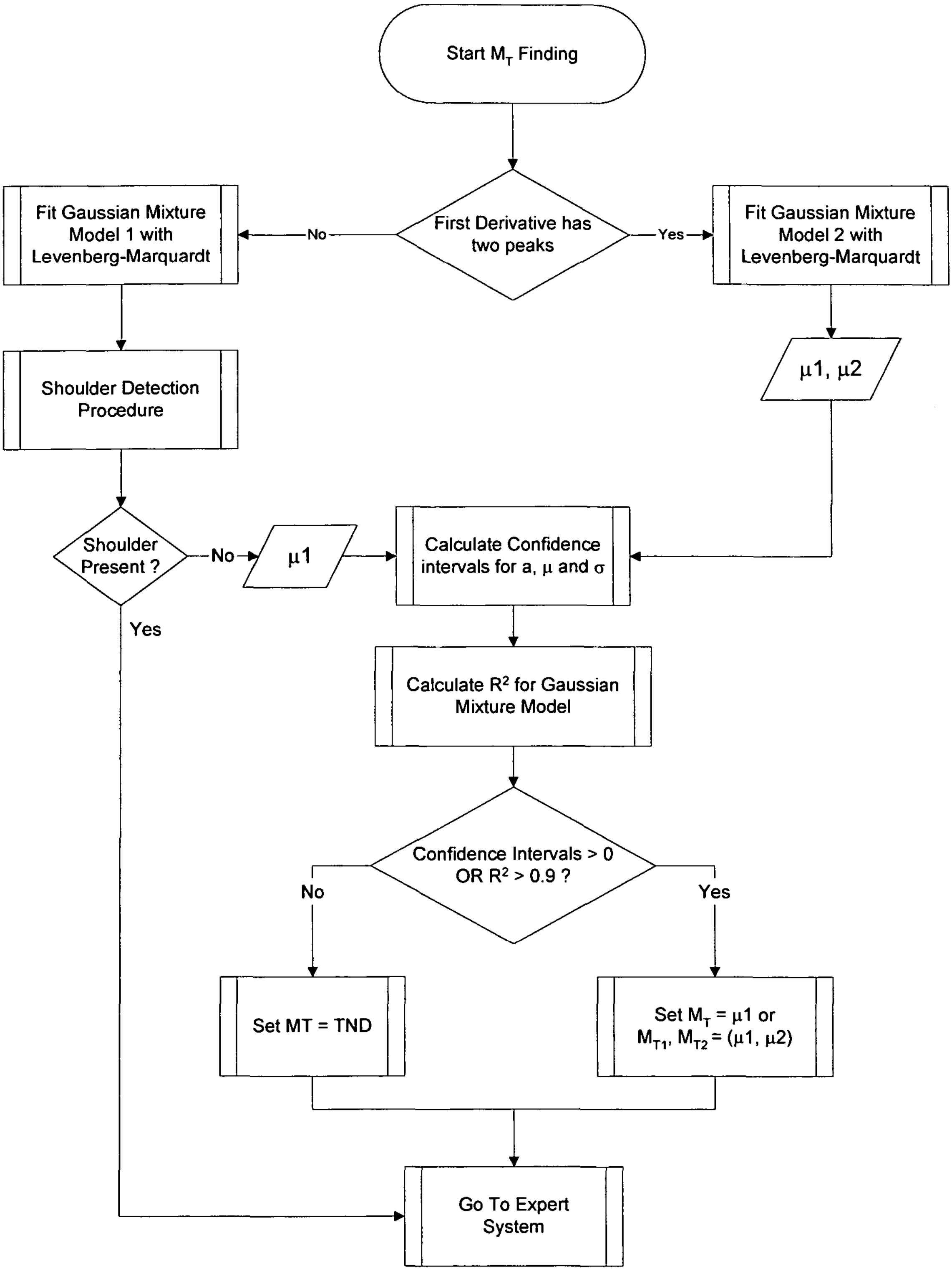


**Figure 5: Major Processing Steps of the Equation-Less Melting Algorithm**

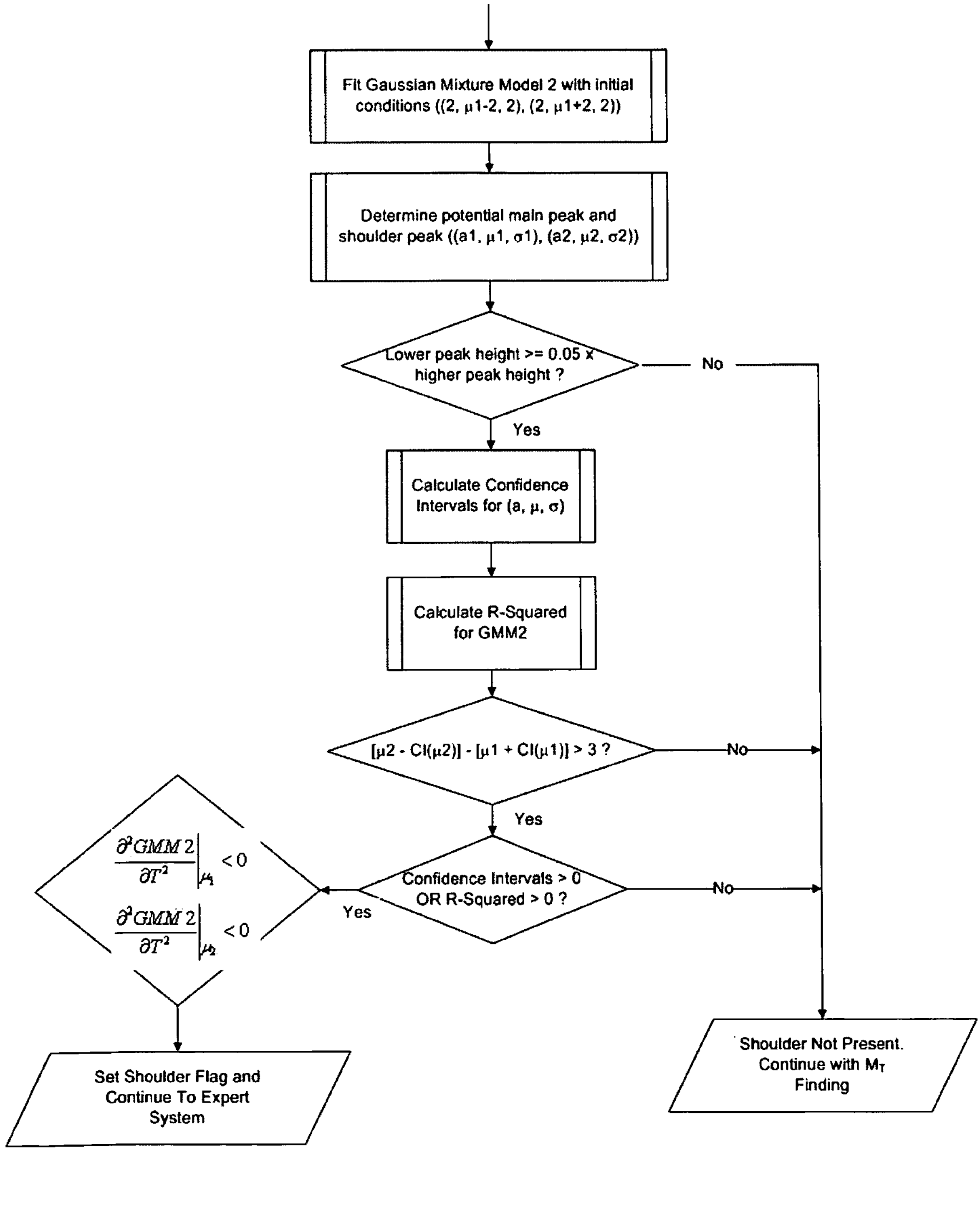


**Figure 6: Major Processing Steps of the Equation-Less Melting Algorithm**

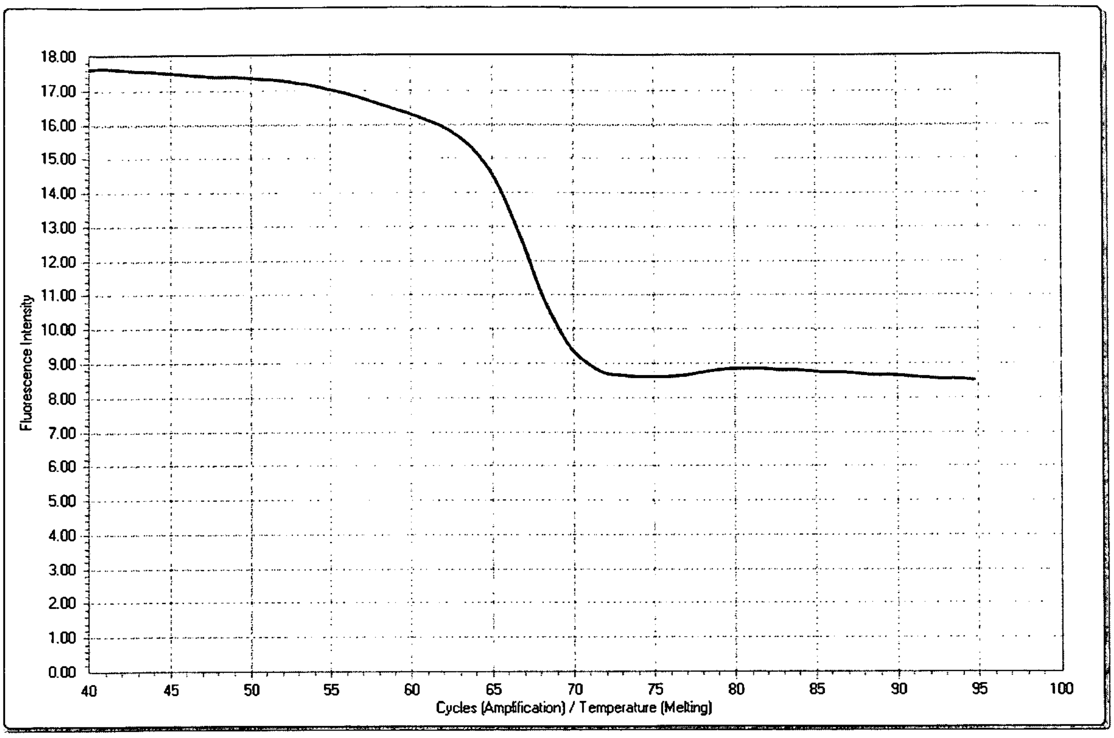


Figure 7a

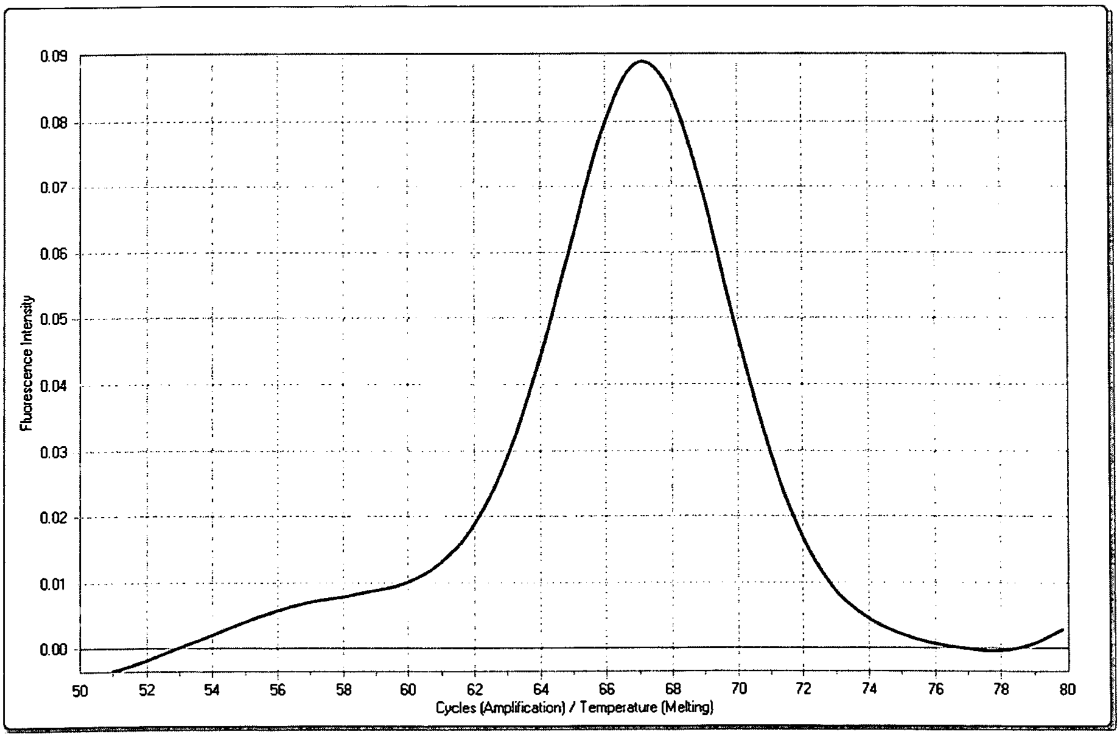


Figure 7b

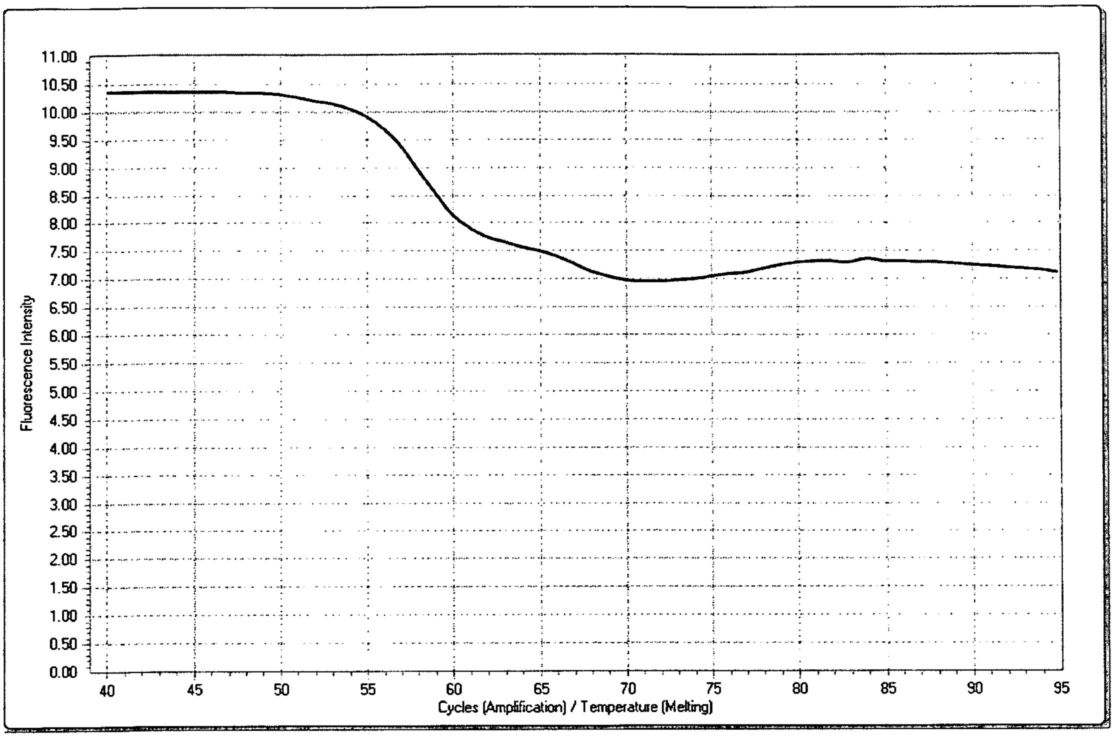


**Figure 8a**

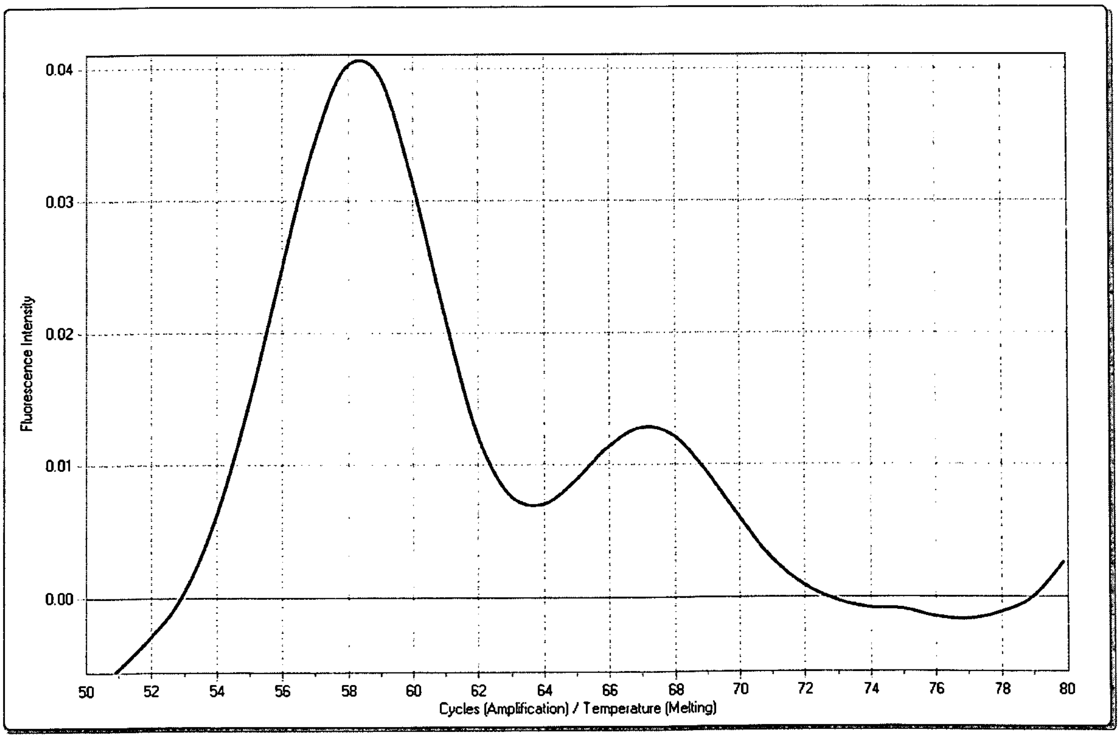


**Figure 8b**

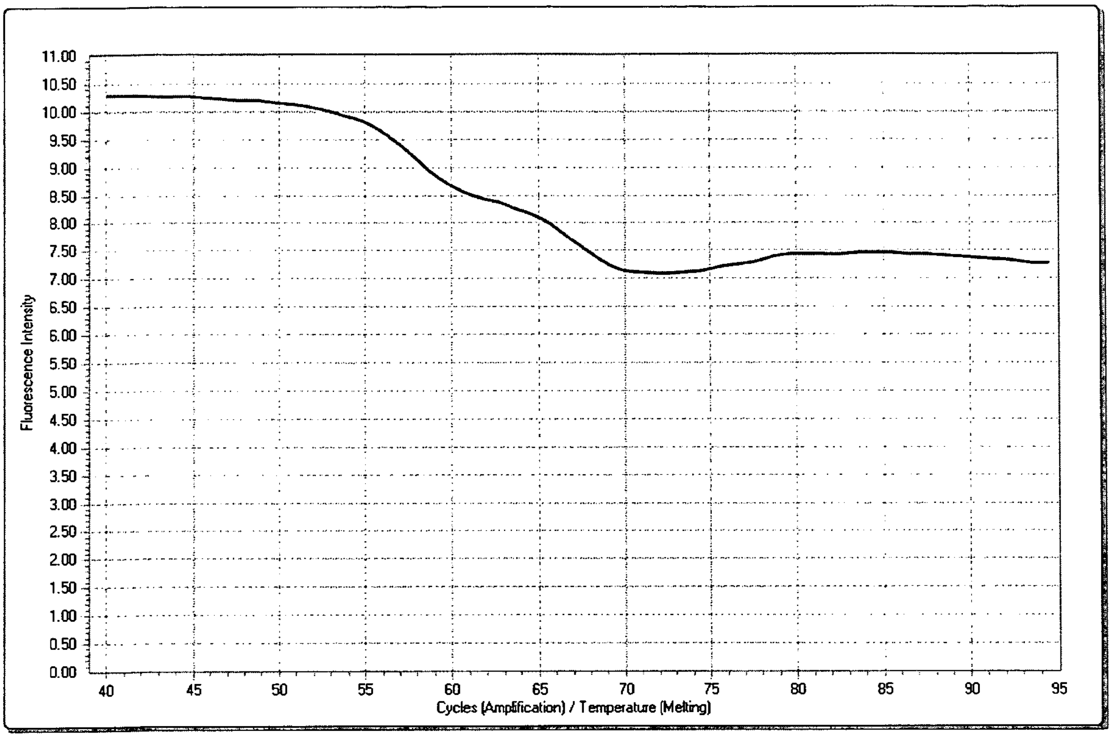


Figure 9a

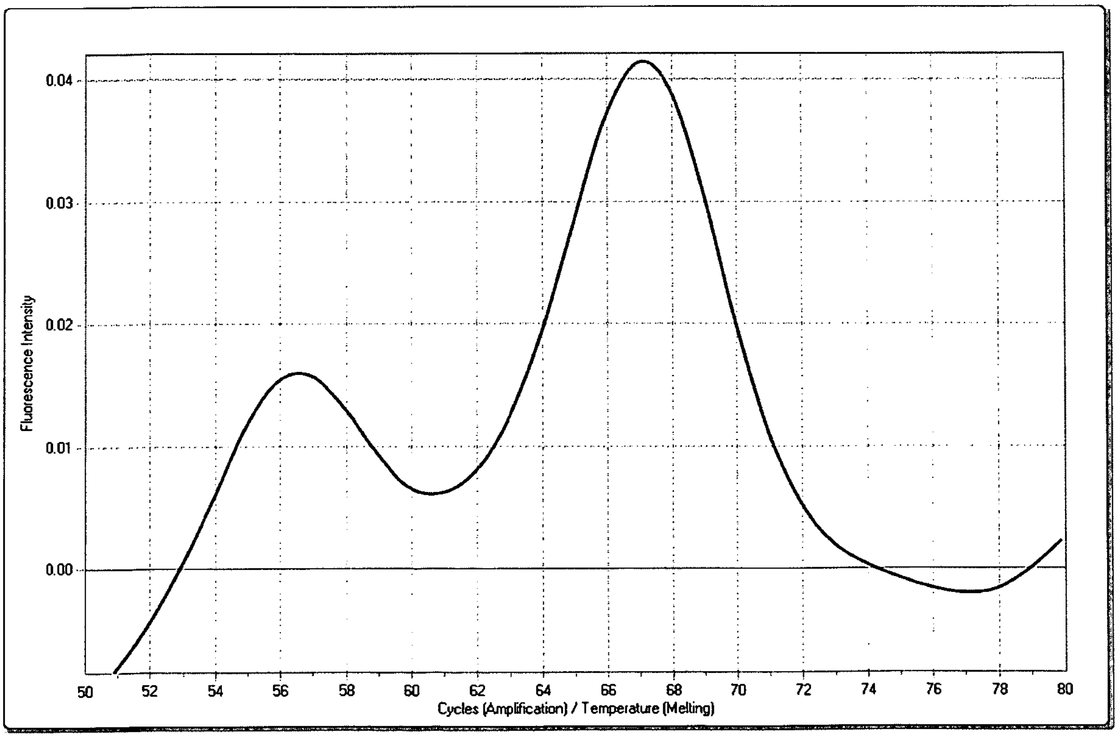


Figure 9b

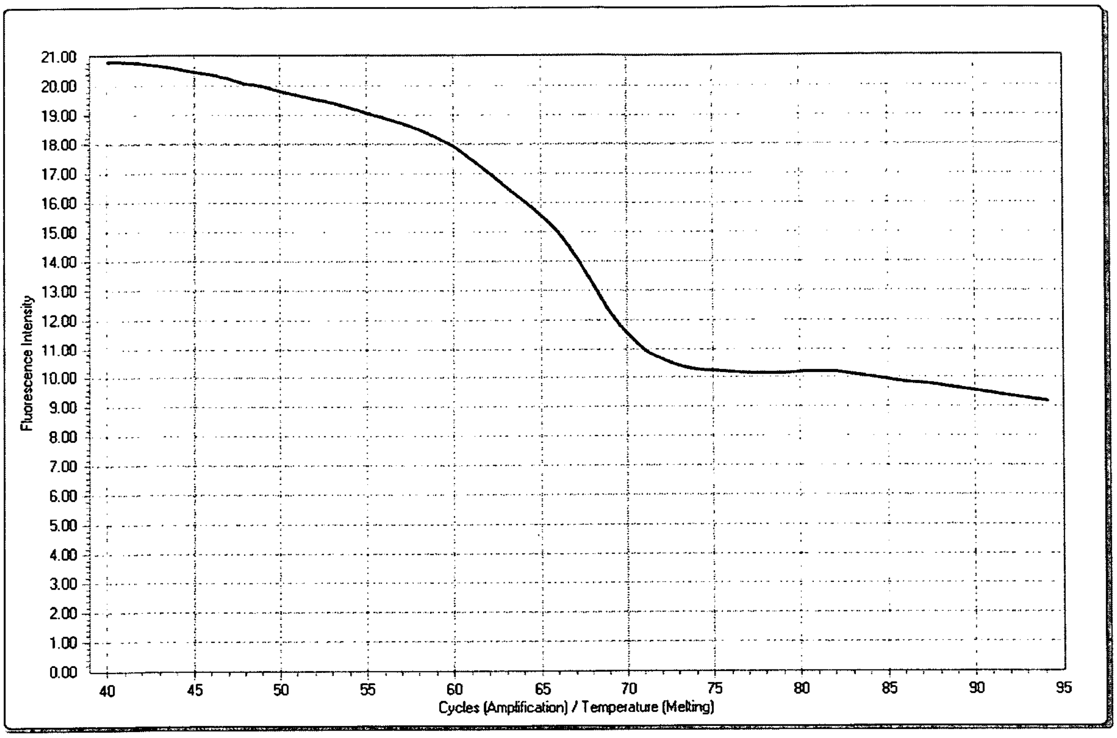


Figure 10a

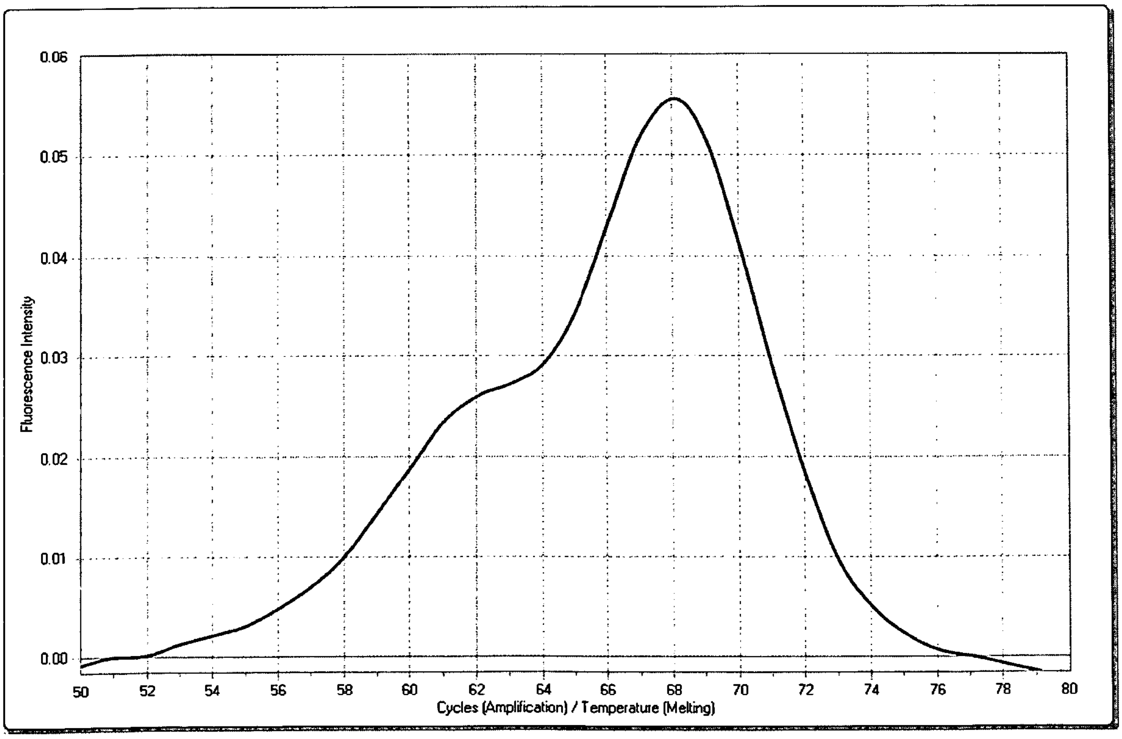


Figure 10b

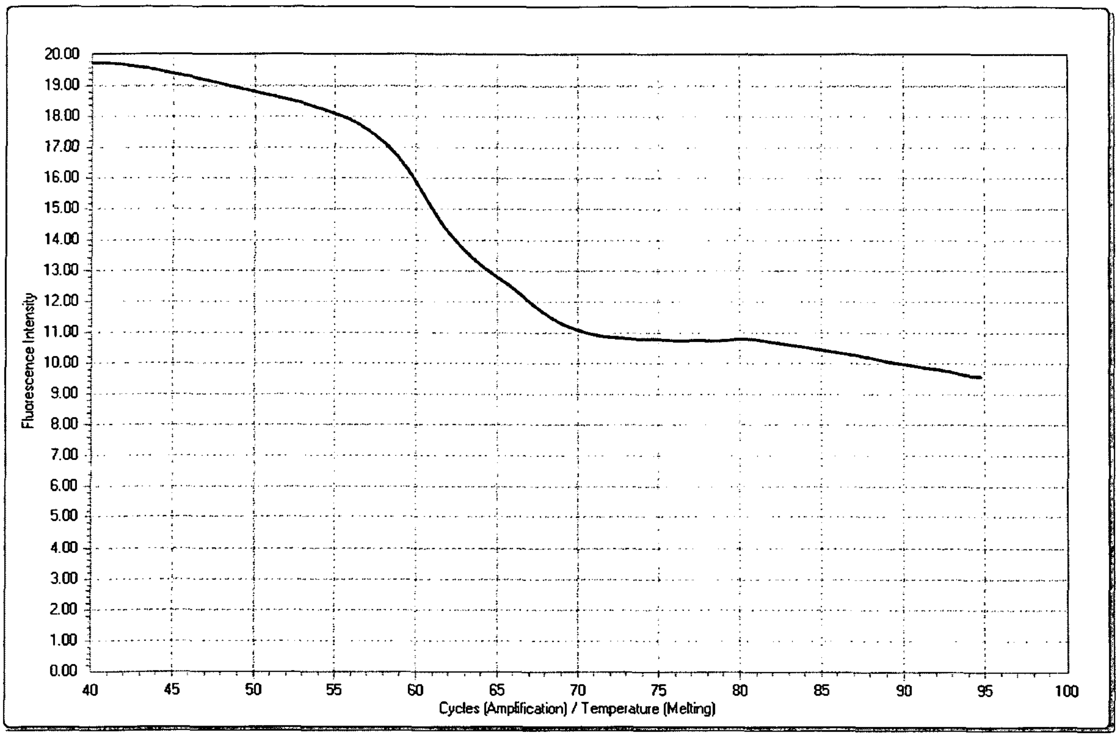


**Figure 11a**

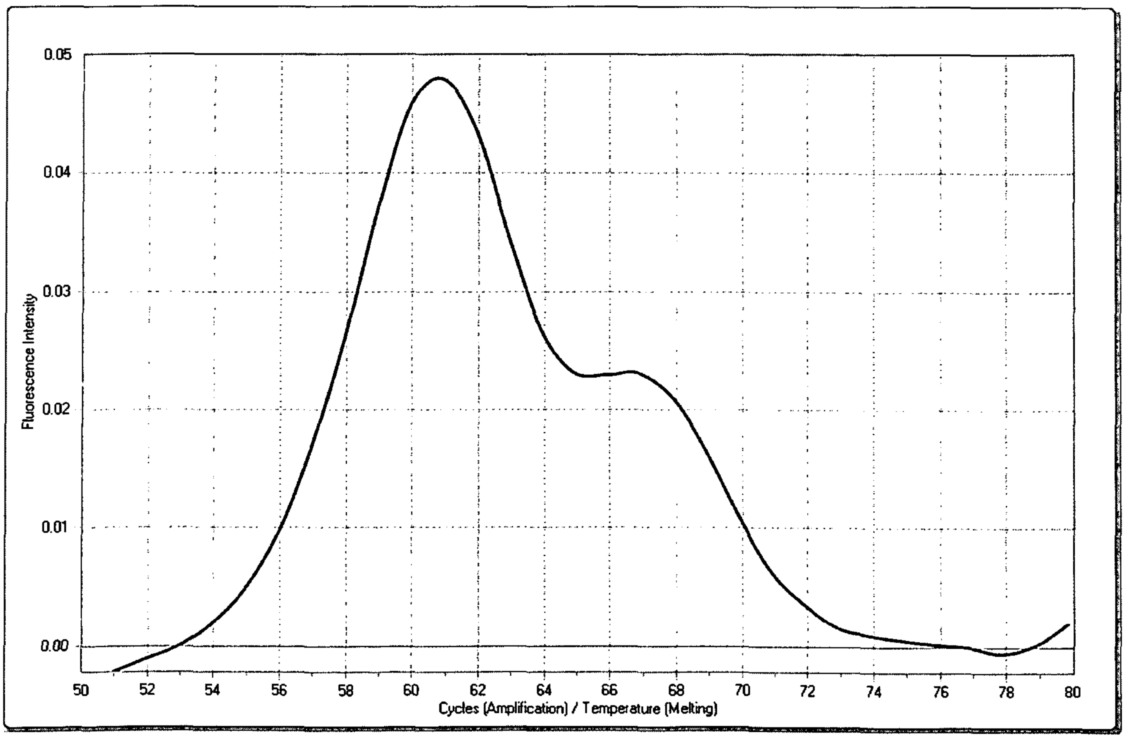


**Figure 11b**

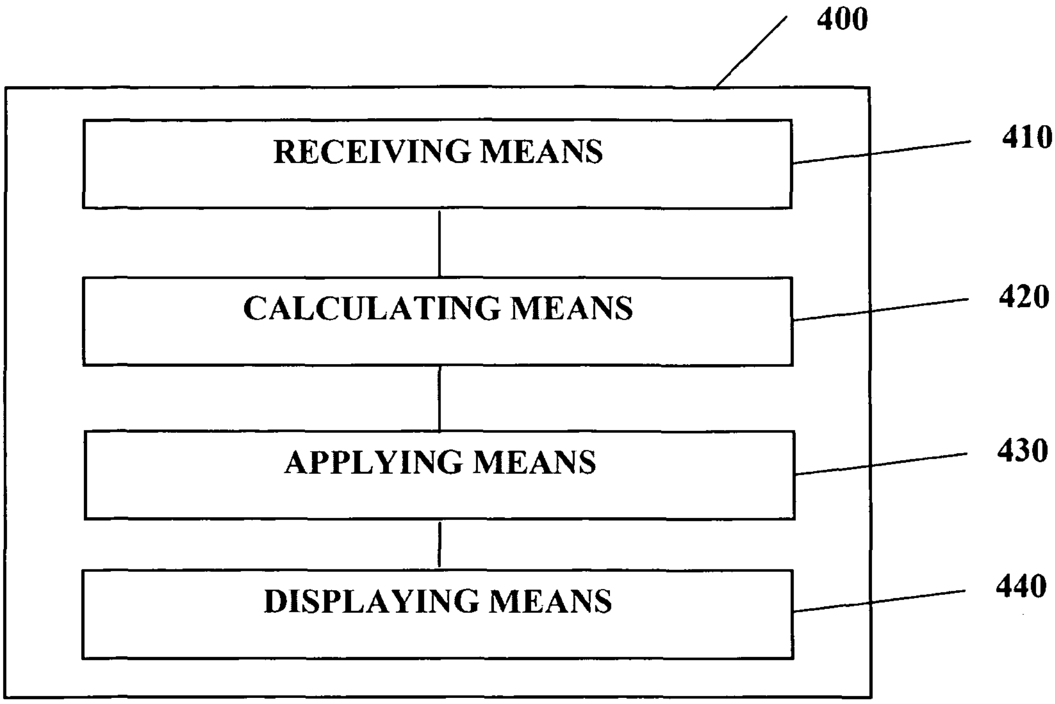
400
RECEIVING MEANS
410
CALCULATING MEANS
420
APPLYING MEANS
430
DISPLAYING MEANS
440

Figure 12

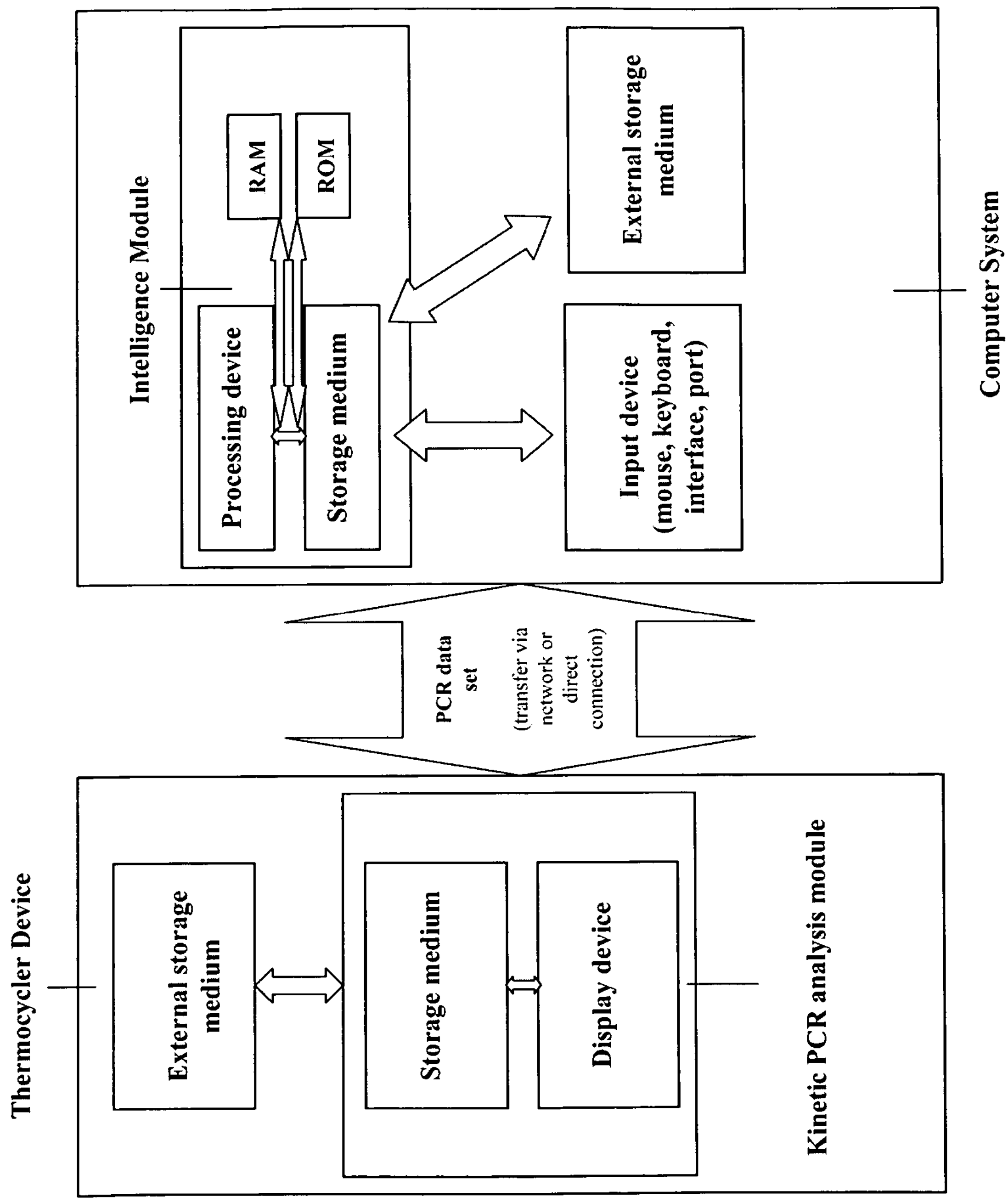
Intelligence Module
RAM
ROM
Processing device
Storage medium
External storage medium
Input device (mouse, keyboard, interface, port)
Computer System
PCR data set
(transfer via network or direct connection)
Thermocycler Device
External storage medium
Storage medium
Display device
Kinetic PCR analysis module

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9746714 A


### Non-patent literature cited in the description


- **A. SAVITZKY ; MARCEL J.E. GOLAY.** Smoothing and Differentiation of Data by Simplified Least Squares Procedures. *Analytical Chemistry,* 1964, vol. 36, 1627-1639
- Numerical Recipes in C. Press, W.H, 650-655
- The Levenberg-Marquardt Algorithm, Implementation and Theory. **MORÉ, J.J.** Numerical Analysis. Springer-Verlag, 1977